# EUROPEAN PATENT SPECIFICATION

(11) **EP 3 716 967 B1**
(45) Date of publication and mention of the grant of the patent: **21.06.2023**
(21) Application number: 18882935.2
(22) Date of filing: 30.11.2018
(51) Int. Cl.: A61K 31/23, A61K 31/232, A61K 38/19, A23L 33/10

(54) **COMPOSITIONS FOR PREVENTION OR TREATMENT OF ACUTE RADIATION SYNDROME**
ZUSAMMENSETZUNGEN ZUR VORBEUGUNG ODER BEHANDLUNG VON AKUTEM STRAHLUNGSSYNDROM
COMPOSITIONS POUR LA PRÉVENTION OU LE TRAITEMENT DU SYNDROME D'IRRADIATION AIGUË

(30) Priority: 30.11.2017 KR 20170162404; 28.06.2018 US 201862691604 P
(43) Date of publication of application: 07.10.2020
(73) Proprietor: Enzychem Lifesciences Corporation, Seoul 137-739 (KR)
(72) Inventor: SOHN, Ki Young, 137-73 (KR); YOON, Sun Young, 137-73 (KR)
(74) Representative: Forstmeyer, Dietmar
(86) International application number: PCT/IB2018/059538
(87) International publication number: WO 2019/106632

(56) References cited:
- WO-A1-2015/176012
- WO-A1-2016/019313
- US-A1- 2017 128 404
- HYUN OK YANG ET AL: "Stimulatory Effects of Monoacetyldiglycerides on Hematopoiesis", BIOLOGICAL & PHARMACEUTICAL BULLETIN, PHARMACEUTICAL SOCIETY OF JAPAN, TOKYO, JP, vol. 27, no. 7, 10 July 2004 (2004-07-10), pages 1121-1125, XP002540177, ISSN: 0918-6158, DOI: 10.1248/BPB.27.1121
- JEONG, J. et al.: "PLAG (1-palmitoyl-2-linoleoyl-3-acetyl-rac-glyc erol) modulates eosinophil chemotaxis by regulating CCL26 expression from epithelial cells", PLOS ONE, vol. 11, no. 3, 24 March 2016 (2016-03-24) , pages 1-14, XP055615960,
- YOON, S. Y. et al.: "1-palmitoyl-2-linoleoyl-3-acetyl-rac-glyc erol ( EC -18) modulates Th2 immunity through attenuation of IL -4 expression", Immune Network, vol. 15, no. 2, April 2015 (2015-04), pages 100-109, XP055354808,

## Description

### FIELD

In one aspect, methods and compositions that comprise PLAG (1-palmitoyl-3-linoleoyl-3-acetylglycerol) are provided for preventing, treating, modulating or mediating acute radiation syndrome (ARS).

### BACKGROUND

Acute radiation syndrome (ARS) is a disease that occurs when a large part of the human body is exposed to radiation; it is a fatal disease that ultimately leads to death by destroying immune, hematopoietic, neurologic and/or gastrointestinal systems. Nuclear explosion and nuclear accident may cause a radiation exposure that can cause the acute radiation syndrome.

A main treatment for patients exposed to radiation is to prevent and manage infection. Clinically, adjuvant therapies such as cytokines, antibiotic administration, and blood transfusions are performed in the early stage after the radiation exposure, and when the duration of neutropenia in patients is prolonged, risk of secondary infection also increases.

It thus would be desirable to have new therapies for acute radiation syndrome. Documents WO2015/176012A1 and Hyun Ok Yang et al: "Stimulatory effects of monoacatyldiglycerides on hematopoisis" in Biological & Pharmaceutical Bulletin, Pharmaceutical Society of japanm vol 27, no. 7, 10 July 2004 disclose the use of PLAG in a medical context.

### SUMMARY

The invention is disclosed in the appended claims.

In one aspect, we now provide new therapies for treatment and prevention of acute radiation syndrome that include PLAG (1-palmitoyl-2-linoleoyl-3-acetylglycerol).

In another aspect, new therapies are provided to treat a subject that has been exposed to ionizing radiation (particularly adverse exposure such as unintended and/or non-therapeutic exposure, and/or exposure to excessive ionizing radiation, including gamma radiation) which include administering to the subject an effective amount of PLAG (1-palmitoyl-2-linoleoyl-3-acetylglycerol).

In a yet further aspect, therapies are provided for treatment and prevention of one or more subsyndromes of acute radiation syndrome that include use of PLAG (1-palmitoyl-2-linoleoyl-3-acetylglycerol). ARS subsyndromes include hematopoietic, gastrointestinal, cutaneous and/or neurovascular.

In a still further aspect, therapies are provided for treatment and prevention of hematopoietic (bone marrow) acute radiation syndrome, gastrointestinal acute radiation syndrome, cutaneous acute radiation syndrome, cardiovascular acute radiation syndrome, and/or central nervous system (CNS) acute radiation syndrome.

In a further aspect, therapies are provided for treatment and prevention of radiation-induced coagulopathy.

PLAG (1-palmitoyl-2-linoleoyl-3-acetylglycerol) can alleviate, prevent and/or treat the reduction of immune cells such as leukocytes, neutrophils and lymphocytes by radiation exposure, and alleviate inflammatory diseases like oral mucositis, and further increase the survival rate of subjects exposed to radiation.

PLAG can be advantangeously first administered to a subject even after an extended period has elapsed since the time the subject was exposed to the injurious radiation such as gamma radiation. That is, the subject can receive a therapeutic benefit, including increased survival times, even after such delayed treatment. See, for instance, the *in vivo* results set forth in the Examples which follow. This can be significant because first treatment of a subject suffering radiation exposure often can be delayed.

Thus, in certain aspects, PLAG is first administered (first dose to a subject) within 3, 6, 12, 18, 24, 36, 48, 60 or 72 hours after the subject suffered an injurious exposure to radiation (including gamma radiation exposure). In particular aspects, PLAG is first administered (first dose to a subject) between 3 and 12, 18, 48 or 72 hours, or between 6 and 18, 24 or 48 hours after the subject suffered an injurious exposure to radiation (including gamma radiation exposure). In certain aspects, an injurious exposure to radiation may be exposure to ionizing radiation such as gamma radiation of 1 Gy to 8 Gy or more for 1 second to 30, 60 or 120 seconds or more.

In a further aspect, a pharmaceutical composition is provided comprising PLAG (1-palmitoyl-2-linoleoyl-3-acetylglycerol) for preventing or treating acute radiation syndrome.

In a yet further aspect, kits are provided for use to treat or prevent acute radiation syndrome, or to treat or prevent exposure to excessive ionizing radiation.

Kits of the invention suitably may comprise 1) 1-palmitoyl-2-linoleoyl-3-acetylglycerol (PLAG); and 2) instructions for using the PLAG for treating or preventing acute radiation syndrome (ARS) of a subject, or for treating or preventing exposure to excessive ionizing radiation. Preferably, a kit will comprise a therapeutically effective amount of PLAG. The instructions suitably may be in written form, including as a product label.

The terms PLAG, EC-18 and 1-palmitoyl-2-linoleoyl-3-acetylglycerol are used interchangeably herein and designate the same compound herein.

In additional aspects, a compound of Formula 1 as set forth below may be employed in the methods, compositons and kits of the invention, particularly to treat a subject suffering from acute radiation syndorme or who have been exposed to adverse ionizing radiation.

Other aspects are disclosed *infra.*

### BRIEF DESCRIPTION OF THE DRAWINGS

FIG. 1 shows a schematic experimental design diagram related to radiation-induced Acute Radiation Syndrome in an animal model.
FIGS. 2A-2C show the level of blood leukocyte (FIG. 2A), neutrophil (FIG. 2B) and lymphocyte (FIG. 2C) counts after PLAG (1-palmitoyl-2-linoleoyl-3-acetylglycerol) administration in an animal model of acute radiation syndrome.
FIG. 3 shows the survival rate with PLAG administration in an animal model with acute radiation syndrome accompanying oral mucositis.
FIG. 4 shows the state of mucositis after PLAG administration in an animal model of acute radiation syndrome.
FIG. 5 shows the calculated mucositis score in PLAG (EC-18)-treated and untreated animals in an animal model of acute radiation syndrome accompanying oral mucositis.
FIGS. 6A-6B show the level of neutrophil counts in the blood following PLAG administration in an animal model of acute radiation syndrome accompanying oral mucositis after 7 days (FIG. 6A) and after 10 days (FIG. 6B).
FIG. 7A is a schematic diagram of EC-18 treatment (30 days) in a radiation-induced Acute Radiation Syndrome animal model. FIG. 7B shows a survival rate of mice (BALB/c, 11 weeks old) administered with or without EC-18 (250 mg/kg/day PO) receiving total body irradiation (6.5 Gy). The survival rate in mice administered with EC-18 is improved compared to the untreated (without EC-18) mice.
FIG. 8 shows a survival rate of mice (BALB/c, 9 weeks old) administered with (n=20) or without (n=20) EC-18 (250 mg/kg/day PO) receiving total body irradiation (6.5 Gy) according to the diagram in FIG. 7A. Survival rate of mice treated with EC-18 is improved compared to the untreated (without EC-18) mice.
FIG. 9 shows survival curves of mice (BALB/c, 9 weeks old) receiving total body irradiation (2, 4, and 6 Gy) without EC-18 treatment and with EC-18 treatment (250 mg/kg/day PO) in mice receiving total body irradiation at 6 Gy according to the diagram in FIG. 7A.
FIG. 10A is a schematic diagram of EC-18 treatment (17 days) in a radiation-induced Acute Radiation Syndrome animal model. FIG. 10B shows survival rate of mice (BALB/c, 9 weeks old) administered with or without EC-18 (250 mg/kg/day PO) receiving lethal today body irradiation (7 Gy). Survival rate of mice administered with EC-18 receiving lethal irradiation is improved until about day 15 compared to untreated (without EC-18) mice.
FIG. 11 shows survival rate of mice (BALB/c, 9 weeks old) administered with or without EC-18 (250 mg/kg/day PO) receiving lethal total body irradiation (8 Gy). Survival rate of mice administered with EC-18 receiving irradiation is improved until about day 14 compared to untreated (without EC-18) mice according to the diagram in FIG. 10A.
FIG. 12A is a schematic diagram of EC-18 treatment (12 days) in a radiation-induced Acute Radiation Syndrome animal model. FIG. 12B shows survival rate of mice (BALB/c, 9 weeks old) administered with or without EC-18 (250 mg/kg/day PO) receiving lethal total body irradiation (10 Gy). Survival rate of mice administered with EC-18 receiving irradiation is improved until about day 9 compared to the untreated (without EC-18) mouse according to the diagram in FIG. 12A.
FIG. 13A is a schematic diagram of EC-18 treatment (17 days) in a radiation-induced Acute Radiation Syndrome animal model. FIG. 13B shows skin erythema in mice (BALB/c, 9 weeks old) administered with and without EC-18 (250 mg/kg/day PO) receiving lethal total body irradiation (8 Gy).
FIG. 14A is a schematic diagram of EC-18 treatment (30 days) in a radiation-induced Acute Radiation Syndrome animal model. FIG. 14B shows skin erythema in mice (BALB/c, 9 weeks old) administrated with or without EC-18 (250 mg/kg/day PO) receiving total body irradiation (6.5 Gy).
FIG. 15 shows skin erythema in mice (BALB/c, 11 weeks old) administrated with or without EC-18 (250 mg/kg/day PO) receiving total body irradiation (6.5 Gy) according to the diagram in FIG. 14A.

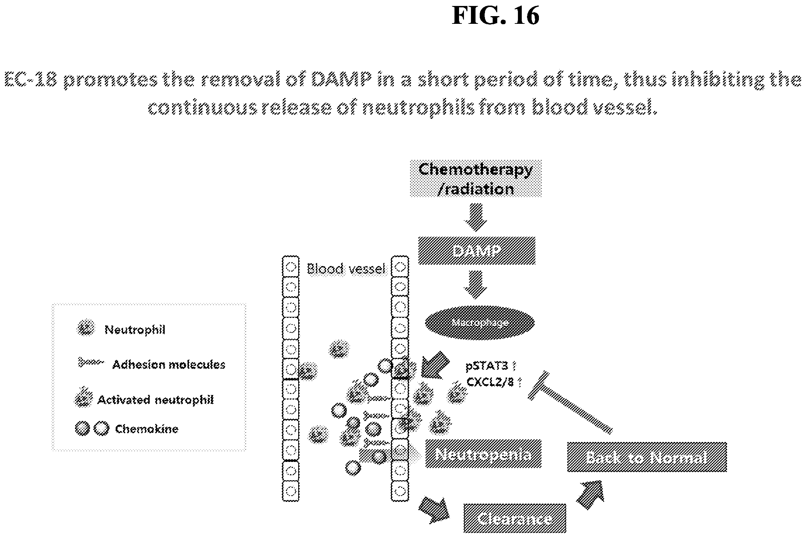

FIG. 16 illustrates a schematic mechanism of action of EC-18 in Radiation Induced Neutropenia (RIN), which illustrates that EC-18 promotes the removal of DAMP in a short period of time, inhibiting the continuous release of neutrophils from blood vessels.
FIG. 17A depicts a schematic diagram of testing the effect of EC-18 on anti-apoptosis in HaCaT Cells. 5-FU and γ-ray-induced damage on HaCaT.
FIG. 17B shows cell number counts in the experiments depicted in FIG. 17A according to 5-FU dose, radiation and treatment with PLAG (EC-18).
FIG. 17C shows early apoptosis rate (%) in the experiments depicted in FIG. 17A according to 5-FU amount, radiation and the treatment with PLAG (EC-18).
FIG. 17D shows early apoptosis rate (%) in the experiments depicted in FIG. 17A 5-at FU amount of 1 ng/mL according to radiation and the treatment with PLAG (EC-18).
FIG. 17E shows early apoptosis rate (%) in the experiments depicted in FIG. 17A 5-at FU amount of 10 ng/mL according to radiation and the treatment with PLAG (EC-18).
FIG. 17F shows early apoptosis rate (%) in the experiments depicted in FIG. 17A 5-at FU amount of 100 ng/mL according to radiation and the treatment with PLAG (EC-18).
FIG. 18A shows late apoptosis rate (%) in the experiments depicted in FIG. 17A according to 5-FU amount, radiation and the treatment with PLAG (EC-18).
FIG. 18B shows late apoptosis rate (%) in the experiments depicted in FIG. 17A 5-at FU amount of 1 ng/mL according to radiation and the treatment with PLAG (EC-18).
FIG. 18C shows late apoptosis rate (%) in the experiments depicted in FIG. 17A 5-at FU amount of 10 ng/mL according to radiation and the treatment with PLAG (EC-18).
FIG. 18D shows late apoptosis rate (%) in the experiments depicted in FIG. 17A 5-at FU amount of 100 ng/mL according to radiation and the treatment with PLAG (EC-18).
FIG. 19A illustrates a schematic diagram of testing the effect of EC-18 on intracellular ROS expressions in HaCaT Cells. 5-FU and γ-ray-induced damage on HaCaT.
FIG. 19B shows ROS measurements in HaCaT Cells exposed to 7 Gy of γ-radiation after treatment of EC-18 after irradiation or without treatment.
FIG. 19C shows cell counts in HaCaT Cells against ROS expression when the cells were exposed to 7 Gy of γ-radiation after treatment of EC-18 after irradiation or without treatment (top: 6 hours after exposure, bottom: 24 hours after exposure).
FIG. 20 illustrates a diagram for an exemplary experiment to test survival rates of treatment of EC-18 (PLAG) on 6.5 Gy of γ-radiation induced Acute Radiation Syndrome animal model (mouse, BALB/c, 11 weeks old).
FIG. 21 shows survival curves for Balb/c mice (n=20 per group) not exposed to total body radiation (control), exposed to 6.5 Gy of γ-radiation (total body; non-treated) and treated with EC-18 beginning at day 1 (24 hours after radiation exposure) or day 2 (48 hours after radiation exposure).
FIG. 22 shows the body weight of Balb/c mice not exposed, exposed to 6.5 Gy of γ-radiation, and exposed to 6.5 Gy of γ-radiation (total body) treated with EC-18 24 or 48 hours after irradiation.
FIG. 23 is illustrates a diagram for an exemplary experiment to test radiation induced coagulopathyin a mouse model.
FIG. 24 depicts a diagram for an exemplary experiment including three groups (5-FU only, 5-FU + PLAG 125 mg/kg, 5-FU + PLAG 250 mg/kg) to check time kinetics of neutrophil in 5-FU (100 mg/kg) induced neutropenia.
FIG. 25 shows *in vitro* experiments comparing the effects of EC-18 against G-CSF by western blot.
FIG. 26 is a table illustrating a comparison EC-18 with G-CSF.
FIG. 27 depicts a diagram of survival time of patients exposed to radiation and various ARS subsyndromes.
FIG. 28A shows survivability of irradiated mice with each γ-radiation dose over 30 days of observation. FIG. 28B shows radiation dose relationship (DRR) using probit models of BALB/c mice exposed to various doses of γ-radiation.
FIG. 29 shows that the normalized body weight changes of irradiated mice exposed to various doses of γ-radiation.
FIGS. 30A-B show therapeutic effect of EC-18 administration in γ-radiation-induced ARS. FIG. 30A shows the survival rate and FIG. 30B shows body weight loss of irradiated mice with a dose of 6.11 Gy of γ-radiation. * p<0.05, **p<0.01, *** p<0.005, for 6.11Gy versus 6.11Gy + EC-18 250 mg/kg.
FIGS. 31A-F are CBC parameters in mice exposed to 6.11Gy of γ-radiation. FIGS. 31A through 31F show the white blood cell (WBC; FIG. 31A), absolute Neutrophil count (ANC; FIG. 31B), monocyte (FIG. 31C), absolute Lymphocyte count (ALC; FIG. 31D), platelet count (PLT; FIG. 31E) and red blood cell count (RBC; FIG. 31F), respectively. n=5 mice/cohort.

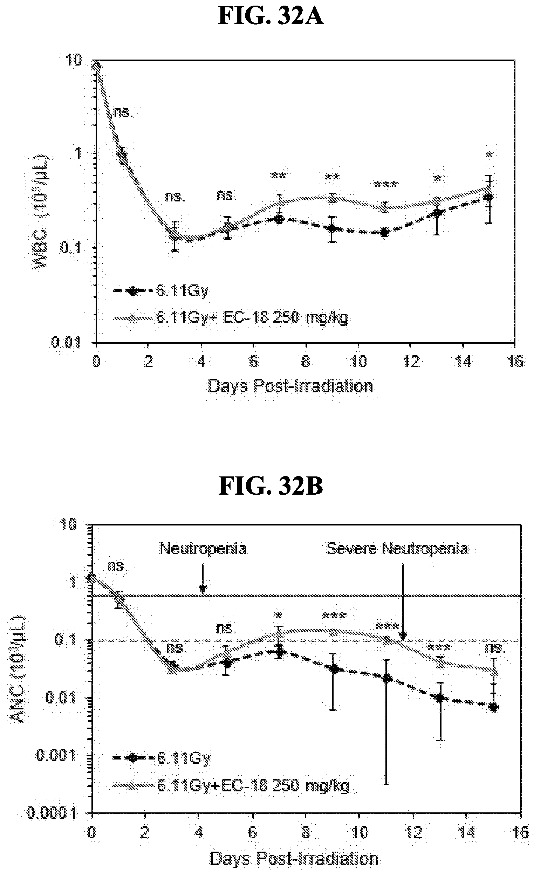

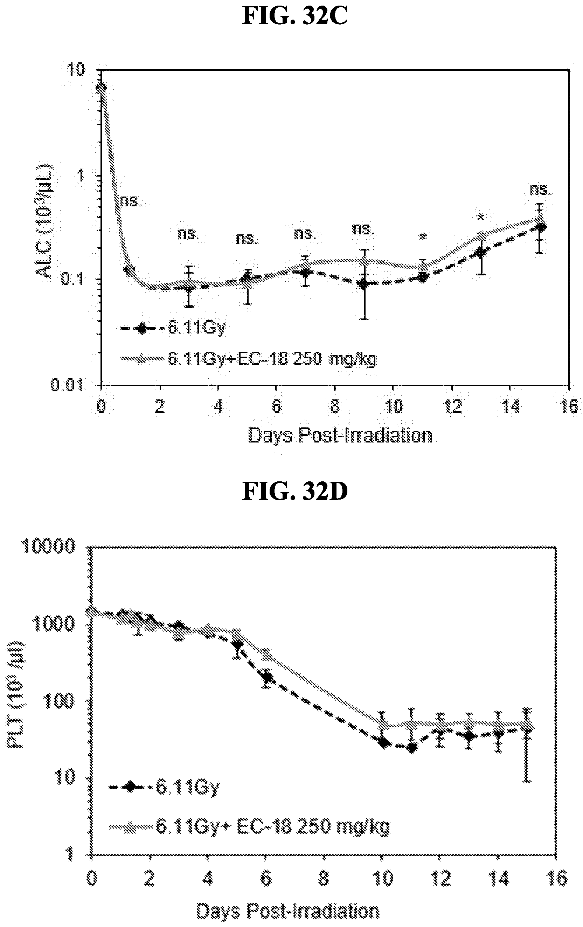

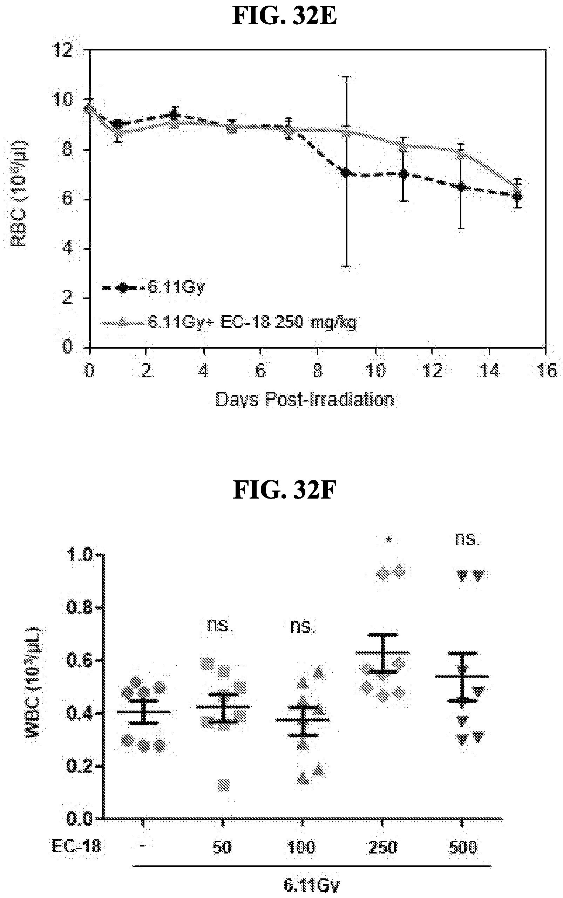

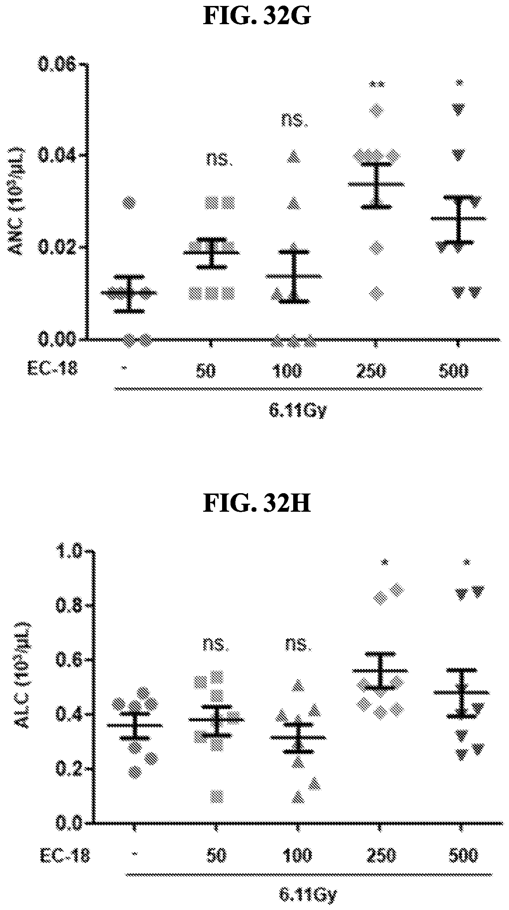

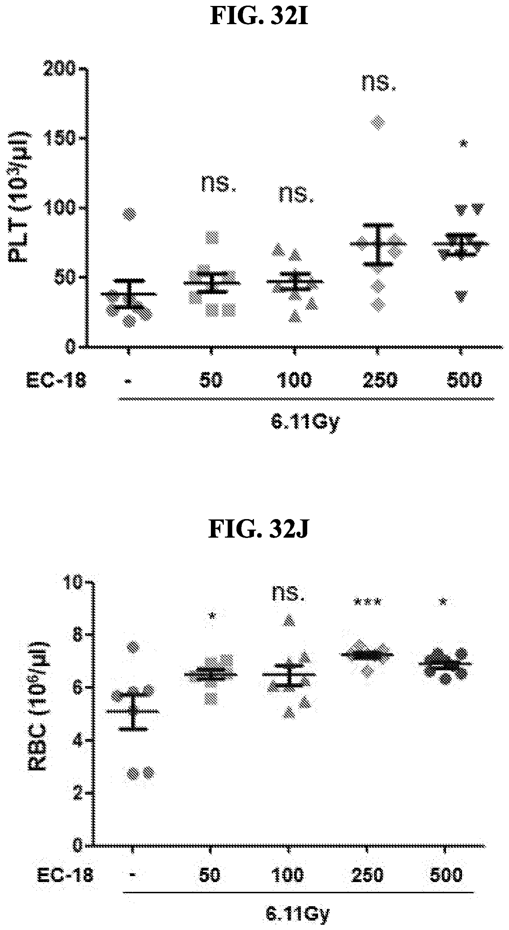

FIGS. 32A-J show therapeutic effect of EC-18 administration on CBC parameters in mice exposed to 6.11Gy of γ-radiation. FIGS. 32A through 32E show the time course of the white blood cell count (WBC; FIG. 32A), absolute Neutrophil count (ANC; FIG. 32B), absolute Lymphocyte count (ALC; FIG. 32C), platelet count (PLT; FIG. 32D) and red blood cell count (RBC; FIG. 32E) over 15 days, respectively. FIGS. 32F through FIG. 32J show the dose effect of EC-18 administration on WBC (FIG. 32F), ANC (FIG. 32G), ALC (FIG. 32H), PLT (FIG. 32I) and BRC (FIG. 32J) on day 15, respectively. ns; not significant, * p<0.05, **p<0.01, *** p<0.005.

### DETAILED DESCRIPTION

### I. Definitions

The abbreviations used herein have their conventional meaning within the chemical and biological arts. The chemical structures and formulae set forth herein are constructed according to the standard rules of chemical valency known in the chemical arts.

As used herein, the term "isomers" refers to compounds having the same number and kind of atoms, and hence the same molecular weight, but differing in respect to the structural arrangement or configuration of the atoms.

The term "tautomer," as used herein, refers to one of two or more structural isomers which exist in equilibrium and which are readily converted from one isomeric form to another.

It will be apparent to one skilled in the art that certain compounds disclosed herein may exist in tautomeric forms, all such tautomeric forms of the compounds being within the scope of the invention.

"Pharmaceutically acceptable excipient" and "pharmaceutically acceptable carrier" refer to a substance that aids the administration of an active agent to and absorption by a subject and can be included in the compositions of the present invention without causing a significant adverse toxicological effect on the patient. Non-limiting examples of pharmaceutically acceptable excipients include water, NaCl, normal saline solutions, lactated Ringer's, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, fatty acid esters, hydroxymethycellulose, polyvinyl pyrrolidine, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the compounds of the invention. One of skill in the art will recognize that other pharmaceutical excipients are useful in the present invention.

"Treating" and "treatment" as used herein include prophylactic treatment. Treatment methods include administering to a subject a therapeutically effective amount of an active agent. The administering step may consist of a single administration or may include a series of administrations. The length of the treatment period depends on a variety of factors, such as the severity of the condition, the age of the patient, the concentration of active agent, the activity of the compositions used in the treatment, or a combination thereof. It will also be appreciated that the effective dosage of an agent used for the treatment or prophylaxis may increase or decrease over the course of a particular treatment or prophylaxis regime. Changes in dosage may result and become apparent by standard diagnostic assays known in the art. In some instances, chronic administration may be required. For example, the compositions are administered to the subject in an amount and for a duration sufficient to treat the patient. The term "treating" and conjugations thereof, may include prevention of an injury, pathology, condition, or disease. In embodiments, treating is preventing. In embodiments, treating does not include preventing.

The term "prevent" refers to a decrease in the occurrence of disease symptoms in a patient. As indicated above, the prevention may be complete (e.g., no detectable symptoms) or partial, such that fewer symptoms are observed than would likely occur absent treatment.

The term "modulate" is used in accordance with its plain ordinary meaning and refers to the act of changing or varying one or more properties. "Modulation" refers to the process of changing or varying one or more properties. For example, a modulator of a disease decreases a symptom, cause, or characteristic of the targeted disease such as ARS and its subsyndromes.

"Patient," "subject," "patient in need thereof," and "subject in need thereof' are herein used interchangeably and refer to a living organism suffering from or prone to a disease or condition that can be treated by administration of a pharmaceutical composition as provided herein. Non-limiting examples include humans, other mammals, bovines, rats, mice, dogs, monkeys, goat, sheep, cows, deer, and other non-mammalian animals. In some embodiments, a patient or subject is human.

An "effective amount" is an amount sufficient for a compound to accomplish a stated purpose relative to the absence of the compound (e.g. achieve the effect for which it is administered, treat a disease, reduce enzyme activity, increase enzyme activity, reduce a catabolic enzyme activity, or reduce one or more symptoms of a disease or condition). An example of an "effective amount" is an amount sufficient to contribute to the treatment, prevention, or reduction of a symptom or symptoms of a disease, which could also be referred to as a "therapeutically effective amount." A "reduction" of a symptom or symptoms (and grammatical equivalents of this phrase) means decreasing of the severity or frequency of the symptom(s), or elimination of the symptom(s). A "prophylactically effective amount" of a drug is an amount of a drug that, when administered to a subject, will have the intended prophylactic effect, e.g., preventing or delaying the onset (or reoccurrence) of an injury, disease, pathology or condition, or reducing the likelihood of the onset (or reoccurrence) of an injury, disease, pathology, or condition, or their symptoms. The full prophylactic effect does not necessarily occur by administration of one dose, and may occur only after administration of a series of doses. Thus, a prophylactically effective amount may be administered in one or more administrations. An "activity decreasing amount," as used herein, refers to an amount of antagonist required to decrease the activity of an enzyme relative to the absence of the antagonist. A "function disrupting amount," as used herein, refers to the amount of antagonist required to disrupt the function of an enzyme or protein relative to the absence of the antagonist. The exact amounts will depend on the purpose of the treatment, and will be ascertainable by one skilled in the art using known techniques (*see, e.g.,* Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

A therapeutically effective amount of PLAG can be initially determined from cell culture assays. Target concentrations will be those concentrations of active compound(s) that are capable of achieving the methods described herein, as measured using the methods described herein or known in the art.

As is well known in the art, therapeutically effective amounts for use in humans can also be determined from animal models. For example, a dose for humans can be formulated to achieve a concentration that has been found to be effective in animals. The dosage in humans can be adjusted by monitoring compounds effectiveness and adjusting the dosage upwards or downwards, as described above. Adjusting the dose to achieve maximal efficacy in humans based on the methods described above and other methods is well within the capabilities of the ordinarily skilled artisan.

The term "therapeutically effective amount" or "effective amount" as used herein, refers to that amount of the therapeutic agent sufficient to ameliorate the disorder, as described above. For example, for the given parameter, a therapeutically effective amount will show an increase or decrease of at least 5%, 10%, 15%, 20%, 25%, 40%, 50%, 60%, 75%, 80%, 90%, or at least 100%. Therapeutic efficacy can also be expressed as "-fold" increase or decrease. For example, a therapeutically effective amount can have at least a 1.2-fold, 1.5-fold, 2-fold, 5-fold, or more effect over a control.

Dosages may be varied depending upon the requirements of the patient and the compound being employed. The dose administered to a patient, in the context of the present invention should be sufficient to effect a beneficial therapeutic response in the patient over time. The size of the dose also will be determined by the existence, nature, and extent of any adverse side-effects. Determination of the proper dosage for a particular situation is within the skill of the practitioner. Generally, treatment is initiated with smaller dosages which are less than the optimum dose of the compound. Thereafter, the dosage is increased by small increments until the optimum effect under circumstances is reached. Dosage amounts and intervals can be adjusted individually to provide levels of the administered compound effective for the particular clinical indication being treated. This will provide a therapeutic regimen that is commensurate with the severity of the individual's disease state.

Utilizing the teachings provided herein, an effective prophylactic or therapeutic treatment regimen can be planned that does not cause substantial toxicity and yet is effective to treat the clinical symptoms demonstrated by the particular patient. This planning should involve the careful choice of active compound by considering factors such as compound potency, relative bioavailability, patient body weight, presence and severity of adverse side effects, preferred mode of administration and the toxicity profile of the selected agent.

"Pharmaceutically acceptable excipient" and "pharmaceutically acceptable carrier" refer to a substance that aids the administration of an active agent to and absorption by a subject and can be included in the compositions of the present invention without causing a significant adverse toxicological effect on the patient. Non-limiting examples of pharmaceutically acceptable excipients include water, NaCl, normal saline solutions, lactated Ringer's, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, fatty acid esters, hydroxymethycellulose, polyvinyl pyrrolidine, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the compounds of the invention. One of skill in the art will recognize that other pharmaceutical excipients are useful in the present invention.

The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

As used herein, the term "administering" means oral administration, administration as a suppository, topical contact, intravenous, intraperitoneal, intramuscular, intralesional, intrathecal, intranasal or subcutaneous administration, or the implantation of a slow-release device, *e.g.,* a mini-osmotic pump, to a subject. Administration is by any route, including parenteral and transmucosal (*e.g*., buccal, sublingual, palatal, gingival, nasal, vaginal, rectal, or transdermal) compatible with the preparation. Parenteral administration includes, *e.g*., intravenous, intramuscular, intra-arteriole, intradermal, subcutaneous, intraperitoneal, intraventricular, and intracranial. Other modes of delivery include, but are not limited to, the use of liposomal formulations, intravenous infusion, transdermal patches, *etc.*

The compositions disclosed herein can be delivered by transdermally, by a topical route, formulated as applicator sticks, solutions, suspensions, emulsions, gels, creams, ointments, pastes, jellies, paints, powders, and aerosols. Oral preparations include tablets, pills, powder, dragees, capsules, liquids, lozenges, cachets, gels, syrups, slurries, suspensions, etc., suitable for ingestion by the patient. Solid form preparations include powders, tablets, pills, capsules, cachets, suppositories, and dispersible granules. Liquid form preparations include solutions, suspensions, and emulsions, for example, water or water/propylene glycol solutions. The compositions of the present invention may additionally include components to provide sustained release and/or comfort. Such components include high molecular weight, anionic mucomimetic polymers, gelling polysaccharides and finely-divided drug carrier substrates. These components are discussed in greater detail in U.S. Pat. Nos. 4,911,920; 5,403,841; 5,212,162; and 4,861,760. The compositions disclosed herein can also be delivered as microspheres for slow release in the body. For example, microspheres can be administered via intradermal injection of drug-containing microspheres, which slowly release subcutaneously (see Rao, J. Biomater Sci. Polym. Ed. 7:623-645, 1995; as biodegradable and injectable gel formulations (see, e.g., Gao Pharm. Res. 12:857-863, 1995); or, as microspheres for oral administration (see, e.g., Eyles, J. Pharm. Pharmacol. 49:669-674, 1997). In another embodiment, the formulations of the compositions of the present invention can be delivered by the use of liposomes which fuse with the cellular membrane or are endocytosed, *i.e.,* by employing receptor ligands attached to the liposome, that bind to surface membrane protein receptors of the cell resulting in endocytosis. By using liposomes, particularly where the liposome surface carries receptor ligands specific for target cells, or are otherwise preferentially directed to a specific organ, one can focus the delivery of the compositions of the present invention into the target cells in vivo. (See, *e.g*., Al-Muhammed, J. Microencapsul. 13:293-306, 1996; Chonn, Curr. Opin. Biotechnol. 6:698-708, 1995; Ostro, Am. J. Hosp. Pharm. 46:1576-1587, 1989). The compositions can also be delivered as nanoparticles.

Pharmaceutical compositions may include compositions wherein the PLAG compound is contained in a therapeutically effective amount, *i.e.,* in an amount effective to achieve its intended purpose. The actual amount effective for a particular application will depend, *inter alia,* on the condition being treated. When administered in methods to treat a disease, such compositions will contain an amount of active ingredient effective to achieve the desired result, e.g., modulating the activity of a target molecule, and/or reducing, eliminating, or slowing the progression of disease symptoms.

The dosage and frequency (single or multiple doses) administered to a mammal can vary depending upon a variety of factors, for example, whether the mammal suffers from another disease, and its route of administration; size, age, sex, health, body weight, body mass index, and diet of the recipient; nature and extent of symptoms of the disease being treated, kind of concurrent treatment, complications from the disease being treated or other health-related problems. Other therapeutic regimens or agents can be used in conjunction with the methods and compounds of Applicants' invention. Adjustment and manipulation of established dosages (e.g., frequency and duration) are well within the ability of those skilled in the art.

"Disease", "disorder" or "condition" refer to a state of being or health status of a patient or subject capable of being treated with the compounds or methods provided herein. As used herein, the term "acute radiation syndrome" or "ARS" refers to a disease or disorder associated with radiation toxicity or radiation sickness (e.g., acute radiation syndrome (ARS) including hematopoietic (bone marrow) acute radiation syndrome, gastrointestinal acute radiation syndrome, cutaneous acute radiation syndrome, cardiovascular acute radiation syndrome, and/or central nervous system (CNS) acute radiation syndrome). The term the term "acute radiation syndrome" or "ARS" also may include radiation-induced coagulopathy. In certain embodiments, the disease is acute radiation syndrome (ARS). In certain embodiments, ARS occurs in a subject upon exposure to a radiation of about 0.1 Gy (or 10 rads) or greater, about 0.2 Gy (or 20 rads) or greater, about 0.3 Gy (or 30 rads) or greater, about 0.4 Gy (or 40 rads) or greater, about 0.5 Gy (or 50 rads) or greater, about 0.6 Gy (or 60 rads) or greater, about 0.7 Gy (or 70 rads) or greater, about 0.8 Gy (or 80 rads) or greater, about 0.9 Gy (or 90 rads) or greater, about 1 Gy (or 100 rads) or greater, about 2.0 Gy(or 200 rads), about 3.0 Gy(or 300 rads) or about 4.0 Gy (or 400 rads) or greater.

In certain embodiments, ARS may occur in a subject upon exposure to radiation (including gamma radiation) of about 1 Gy (or 100 rads) to about 8 Gy (or 800 rads) for any varying time periods such as at least 1, 2, 5, 10, 30, 60, 80, 120, 180, 240 or 300 seconds.

In certain embodiments, the methods and compositions disclosed herein to treat a subject that has been exposed to ionizing radiation (particularly exposure to excessive ionizing radiation, including gamma radiation) -- which methods and compositions may include use or administering to a subject an effective amount of PLAG (1-palmitoyl-2-linoleoyl-3-acetylglycerol) -- are suitably utilized where the subject has been exposed to radiation of about 1 Gy (or 100 rads) to about 8 Gy (or 800 rads) or more for any varying time periods such as at least 1, 2, 5, 10, 30, 60, 80, 120, 180, 240 or 300 seconds.

In certain embodiments, the disease or disorder includes cutaneous radiation syndrome such as skin damages, erythema, altered sensation, itching, edema, blistering, desquamation, ulcer, necrosis, hair loss, onycholysis, and the like. In certain embodiments, the disease or disorder includes neutrophil, or infection by reduced white blood cells. In certain embodiments, the disease or disorder includes hemorrhage. In certain embodiments, the disease or disorder includes diarrhea. In certain embodiments, the disease or disorder includes dehydration or electrolyte imbalance. In certain embodiments, the disease or disorder includes convulsion and/or coma.

As discussed, in certain embodiments, methods and compositions as disclosed herein are used to treat a subject that has been exposed to ionizing radiation. In particular aspects, the radiation exposure may be unintended or accidental. In additional aspects, the radiation exposure will not be for therapeutic purposes, for example the radition exposure will not be radiotherapy as may be utilized for treatment of cancer or other therapy. In further aspects, at least a substantial portion (e.g. an entire limb and/or torso and/or entire head region) of a subject may be exposed to the radiation.

As referred to herein, together and any one of such unintended, accidential, non-therapeutic, and/or substantial body portion exposure may be referred to as "adverse" radiation exposure.

### II. Compositions

As discussed, one aspect of the present invention provides a therapeutic pharmaceutical composition for use in preventing or treating Acute Radiation Syndrome comprising PLAG (1-palmitoyl-2-linoleoyl-3-acetylglycerol) (also referred to herein as EC-18) as an active ingredient.

As referred to herein, the term "acute radiation syndrome" is an acute disease caused by radiation exposure on the entire or on a substantial portion of the body; acute radiation syndrome is also known as radiation toxicity or radiation sickness. Radiation is the released energy when an unstable nucleus is converted into another nucleus. When radiation passes through the body, radiation energy is absorbed and can cause ionization in the tissue. At this time, H₂O is ionized and can transformation of DNA of the subject.

Clinical characteristics of acute radiation syndrome include hematopoietic syndrome, gastrointestinal syndrome, cardiovascular syndrome and neurovascular syndrome.

As described above, acute radiation syndrome can damage radiation-sensitive systems, such as immune, hematopoietic, and gastrointestinal systems, and lead to death. Therefore, the most important factor to consider for preventive or therapeutic effect on acute radiation syndrome is the increase of survival rate of the subjects exposed to radiation. In the present invention, the acute radiation syndrome may be any of the symptoms of hematopoietic syndromes, gastrointestinal syndromes, cardiovascular syndromes and neurovascular syndromes, but at the same time the acute radiation syndrome is not limited to the above symptoms. The composition from this invention is effective in preventing, treating, or mediating the above symptoms and ultimately improves the survival rate of the subjects exposed to radiation.

Acute radiation syndrome usually progresses with four clinical stages: prodromal phase, latent phase, manifest phase, and recovery or death. Depending on the amount of radiation absorbed, the symptoms may appear within hours to weeks. The prodromal phase usually starts within 48 hours after radiation exposure, but may last up to 6 days after the exposure and symptoms may include nausea, vomiting, fatigue, autonomic nerve anxiety and loss of consciousness. The latent phase may last from several days to several weeks depending on the amount of radiation exposure, and clinical symptoms may not appear partially or completely. However, at this stage, symptoms such as lymphocytopenia, granulocytopenia, and myelogenous deficiency may occur. Symptoms from the manifest phase may occur with several weeks delay. Symptoms from the manifest phase may include hematopoietic syndrome, gastrointestinal syndrome, cardiovascular syndrome, and neurovascular syndrome depending on the amount of radiation exposure. Patients exposed to extreme amounts of radiation can experience all four of these steps within a few hours and die within a short period of time.

Among the acute radiation syndrome, the hematopoietic syndrome may be affected, induced or caused by radiation dose of about 0.7 - 10 Gy, gastrointestinal syndrome may be affected, induced or caused by radiation dose of about 10 - 30 Gy, and cardiovascular / neurovascular syndrome may be affected, induced or caused by radiation dose of about 50 Gy. In exemplary embodiments of the present invention, acute radiation syndrome includes syndromes affected, induced or caused, by radiation dose of about 0.1 to 100 Gy, about 0.1 to 80 Gy, about 0.1 to 70 Gy, about 0.1 to 60 Gy, about 0.1 to 50 Gy or about 0.7 or 50 Gy, and may be affected, induced or caused by radiation dose of about 0.1 Gy, about 0.5 Gy, about 0.7 Gy, about 1.0 Gy, about 2.0 Gy, about 3.0 Gy or about 4.0 Gy or greater, which may cause death in a specially exposed subject (Reports of Practical Oncology and Radiotherapy, 2011, 16 (4): 123-130), but is not limited thereto.

In additional exemplary embodiments, acute radiation syndrome includes syndromes affected, induced or caused, by radiation dose of about 1 Gy (or 100 rads) to about 8 Gy (or 800 rads) for any varying time periods such as at least 1, 2, 5, 10, 30, 60, 80, 120, 180, 240 or 300 seconds.

The term "prevention" as used herein means any action that inhibits or delays the occurrence, spread or reoccurrence of a specified disorder or disease such as acute radition syndrome upon administration of a compound or composition as disclosed herein. The term "treatment" as used herein means any act that improves or alleviates the symptoms of a specified disorder or disease such as acute radition syndrome upon administration of a compound or composition as disclosed herein.

Disclosed herein is a compound of the following Formula 1: 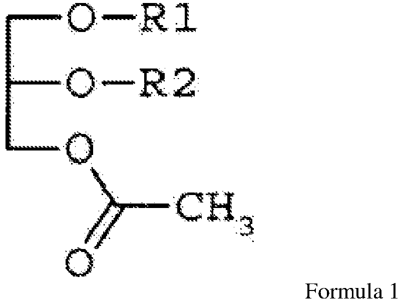 wherein R₁ and R₂ are independently a fatty acid residue of 14 to 20 carbon atoms.

The glycerol derivatives of Formula I above are sometimes referred to herein as monoacetyldiacylglycerols (MDAG). Fatty acid residue refers to the acyl moiety resulting from formation of an ester bond by reaction of a fatty acid and an alcohol. Non-limiting examples of R₁ and R₂ thus include palmitoyl, oleoyl, linoleoyl, linolenoyl, stearoyl, myristoyl, arachidonoyl, and so on. Preferable combinations of R₁ and R₂ (R₁/R₂) include oleoyl/palmitoyl, palmitoyl/oleoyl, palmitoyl/linoleoyl, palmitoyl/linolenoyl, palmitoyl/arachidonoyl, palmitoyl/stearoyl, palmitoyl/palmitoyl, oleoyl/stearoyl, linoleoyl/palmitoyl, linoleoyllstearoyl, stearoyl/linoleoyl, stearoyl/oleoyl, myristoyl/linoleoyl, myristoyl/oleoyl, and so on. In optical activity, the monoacetyldiacylglycerol derivatives of Formula 1 can be (R)-form, (S)-form or a racemic mixture, and may include their stereoisomers. In certain aspects, in compounds where R₁ and/or R₂ substituents are unsaturated fatty acid residues, one or more double bonds that are present suitably may have the cis configuration. In other aspects, one or more double bonds R₁ and/or R₂ substituents may be present in a trans configuration. In certain aspects, such one or more double bonds will be present only in cis configuration.

The compound administered to a subject is PLAG (1-palmitoyl-2-linoleoyl-3-acetylglycoerol) having a structure of the following Chemical Formula 2 (the compound also sometimes referred to herein as EC-18):

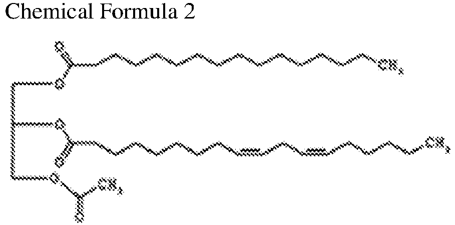

PLAG has been known to have therapeutic effects on neutropenia, thrombocytopenia, and mucositis caused by anti-cancer chemotherapy. However, efficacy of PLAG on preventing or treating acute radiation syndrome, including whether it can increase the survival rate of individuals with acute radiation syndrome has not been reported.

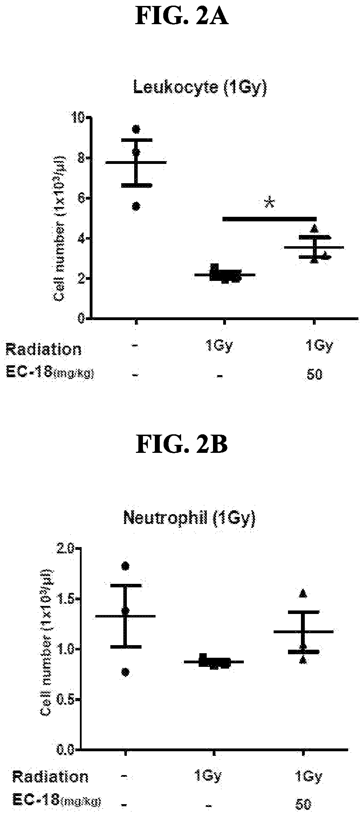

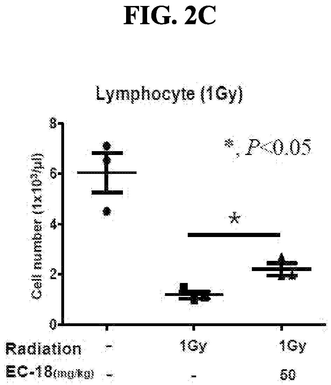

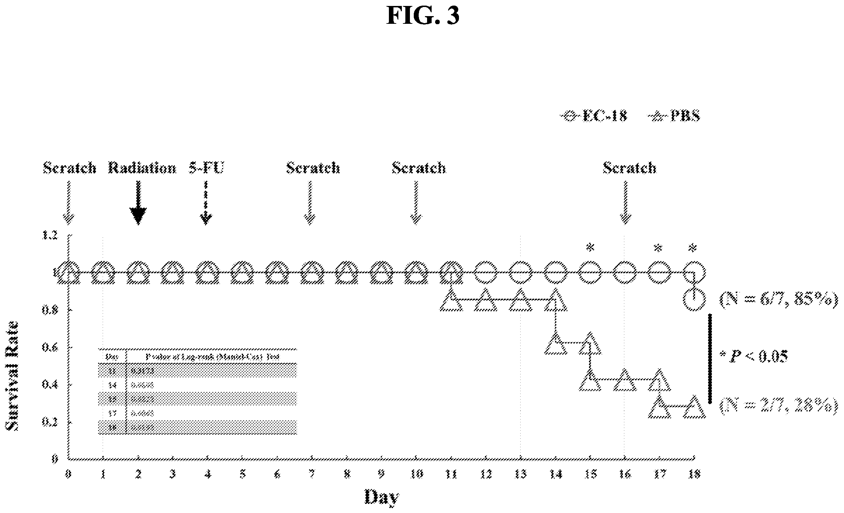

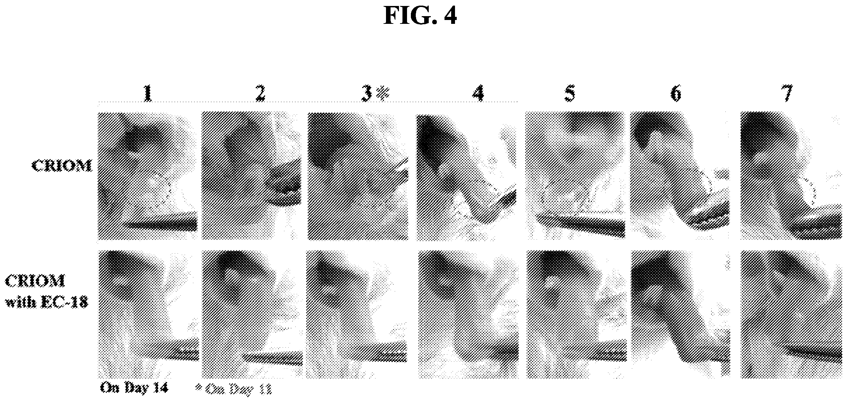

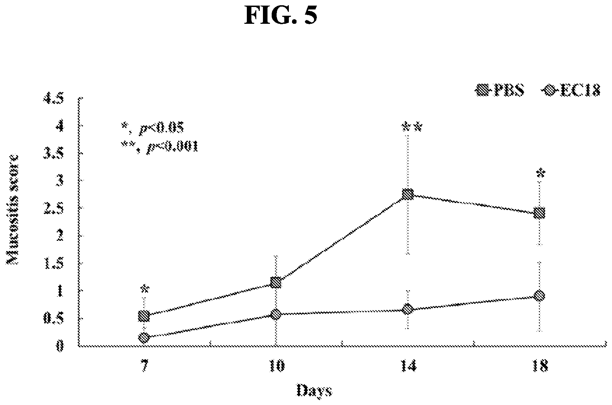

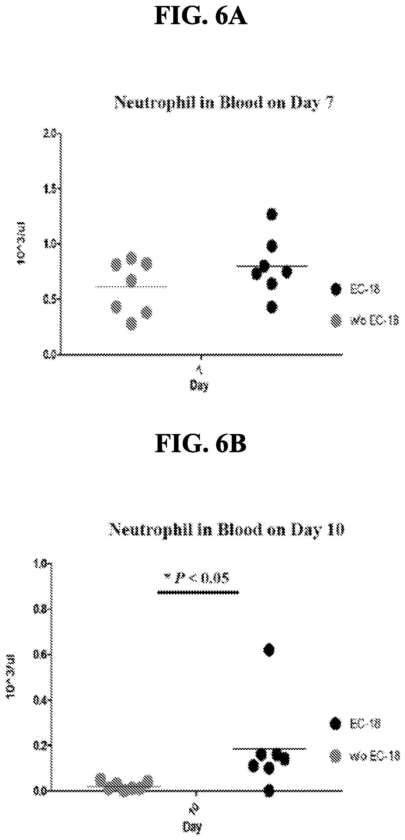

In an exemplary embodiment, PLAG was found to significantly increase the numbers of leukocyte, neutrophil and lymphocyte counts in blood of subjects with radiation exposure (Table 1 and FIG. 2), and improved mucositis from the animal study model with severe acute radiation syndrome where oral mucositis was induced from the chemoradiotheraphy (FIGS. 4 and 5) and increased neutrophils in the blood (FIG. 6), and as a result PLAG dramatically increased the survival rate of the study subjects (FIG. 3). Therefore, the composition of the present invention has an excellent effect as a therapeutic pharmaceutical composition for preventing or treating acute radiation syndrome.

The therapeutic pharmaceutical composition of the present invention may be administered within 1 hour, within 2 hours, within 3 hours, within 4 hours, within 5 hours, within 6 hours, within 7 hours, within 8 hours, within 9 hours, within 10 hours, within 12 hours, within 16 hours, within 20 hours, within 30 hours, within 40 hours, or within 48 hours after the radiation exposure, but is not limited thereto, as discussed above.

Also, the above composition may be administered as an individual therapeutic agent or may be administered in combination with another drug that is known to have an efficacy on treating acute radiation syndrome. For example, the above composition may be administered with one or more of therapeutic agents including proteins, small molecule drugs, nucleic acids or the like. For example, the composition may be administered with a therapeutic agent including granulocyte-colony stimulating factor (G-CSF), but the administration is not limited thereto. Further, the above composition can be administered together with analgesics, anti-ulcer agents, antidiarrheic, antibiotics, antipyretics, nutritional supplements and antioxidants, which can help preventing or treating acute radiation syndrome.

The term "administration" in the present invention means introducing a therapeutic pharmaceutical composition of the present invention to a patient by any suitable method, and the administration route of the composition of the present invention may be administered via various routes whether orally or non-orally. The therapeutic pharmaceutical composition of the present invention can be manufactured into various formulations depending on the administration methods.

The frequency of administration of the composition of the present invention is not particularly limited, but it may be administered once a day or several times a day with divided dosage.

The therapeutic pharmaceutical composition of the present invention can be used as a single medication, and can be used as a combined medication containing another drug, and can be formulated with using a pharmaceutically acceptable carrier, excipient or diluent to make a single-dose unit or a unit with a multi-dose container.

The term "pharmaceutical composition" as referred to herein indicates a composition prepared for the purpose of preventing or treating diseases, and can be formulated into various forms according to ordinary methods. For example, it can be formulated into oral administration formulations such as powders, granules, tablets, capsules, suspensions, emulsions and syrups, and can be formulated in the form of external use, suppositories, and sterilized injection solutions.

In addition, the pharmaceutical composition of the present invention may be manufactured with additional pharmaceutically acceptable carrier for each formulation. As used herein, the term "pharmaceutically acceptable carrier" may refer to a carrier or diluent that does not stimulate organism and not inhibiting biological activity and characteristic of the injected compound. The type of the carrier that can be used in the present invention is not particularly limited, any carrier conventionally used in the area of industry and pharmaceutically acceptable may be used.

Saline, sterilized water, IV fluids, buffer saline, albumin injection solution, dextrose solution, maltodextrin solution, glycerol, ethanol are non-limiting examples of the usable carriers. These carriers may be used alone or in combination of two or more. The carrier may include a non-naturally occurring carrier. If necessary, other conventionally used additives like an antioxidant, a buffer and / or a bacteriostatic agent may be added and used. It may be formulated with diluent, a dispersant, a surfactant, a bonding agent, a lubricant to make an injection solution like aqueous solution, suspension, emulsion, and pills, capsules, granules or tablets, and the like.

In addition, the pharmaceutical composition of the present invention may contain a pharmaceutically effective amount of PLAG. The term "pharmaceutically effective amount" in the present invention means an amount sufficient to treat a disease at a reasonable benefit or risk ratio applicable to medical treatment and is generally in the range of about 0.001 to 5000 mg/kg, preferably of about 0.05 to 1000 mg/kg, may be administered once a day or several times a day with divided dosage. However, for the purposes of the present invention, the specific therapeutically effective amount for a particular patient will depend upon the nature and extent of the reaction to be achieved, the particular composition, including whether or not other agents are used, the age, weight, sex and diet of the patient, the time of administration, the route of administration and the proportion of the composition, the duration of the treatment, the drugs administered or co-administered with the specific composition, and similar compounds well known in the medical industry.

As discussed, kits are also provided. For instance, in this aspect, a PLAG compound suitably can be packaged in suitable containers labeled, for example, for use as a therapy to treat a subject suffering from acute radiation sickness, or a subsyndrome thereof, or exposure to excessive ionizing (e.g. gamma) radiation. The containers can include a PLAG compound or composition and one or more of a suitable stabilizer, carrier molecule and/or the like, as appropriate for the intended use. In other embodiments, the kit further comprises one or more therapeutic reagents that alleviate some of the symptoms or secondary infections or disorders that may be associated with acute radiation sickness, or a subsyndrome thereof, or exposure to excessive ionizing (e.g. gamma) radiation. Accordingly, packaged products (e.g., sterile containers containing one or more of the compositions described herein and packaged for storage, shipment, or sale at concentrated or ready-to-use concentrations) and kits, including a PLAG compound, and instructions for use, are also within the scope of the invention. A product can include a container (e.g., a vial, jar, bottle, bag, or the like) containing a PLAG compound or composition. In addition, an article of manufacture or kit further may include, for example, packaging materials, instructions for use, syringes, delivery devices, for treating or monitoring the condition for which prophylaxis or treatment is required.

The product may also include a legend (e.g., a printed label or insert or other medium describing the product's use (e.g., an audio- or videotape)). The legend can be associated with the container (e.g., affixed to the container) and can describe the manner in which the compositions therein should be administered (e.g., the frequency and route of administration), indications therefor, and other uses. The compositions can be ready for administration (e.g., present in dose-appropriate units), and may include one or more additional pharmaceutically acceptable adjuvants, carriers or other diluents and/or an additional therapeutic agent. Alternatively, the compositions for example can be provided in a concentrated form with a diluent and instructions for dilution.

Described herein (but not claimed) is a health functional food composition of food supplement for preventing or ameliorating Acute Radiation Syndrome comprising PLAG (1-palmitoyl-2-linoleoyl-3-acetylglycerol) as an active ingredient.

In the present invention, the term "improvement" means all actions that at least reduce the degree of symptom associated with the condition being treated. Herein, the health functional food composition may be used simultaneously or separately with the medicament for treatment before or after the occurrence of the disease to prevent or improve the acute radiation syndrome.

PLAG does not show notable toxicity to cells and shows improvement effect on acute radiation syndrome, so PLAG can be manufactured and be taken in the form of a health functional food composition.

Functional food is the same term as food for special health use (FoSHU). It refers to foods that have been processed so that the biological control function appears more efficient in addition to nutritional value. The food may be prepared in various forms such as tablets, capsules, powders, granules, liquids, rings and the like in order to obtain a useful effect on skin regeneration.

For that, the content level of PLAG contained in the health functional food is not particularly limited, but may be 0.01 to 100% by weight, specifically 1 to 80% by weight based on the total weight of the health functional food.

The health functional food composition described herein may also contain a pharmaceutically acceptable carrier.

There is no particular limitation on the kind of health functional foods including PLAG and examples thereof include drinks, gums, tea, vitamin complex, health supplement foods and the like. The food may be supplemented with other ingredients that do not interfere with the improvement effect on acute radiation syndrome, and the kind thereof is not particularly limited. For example, various herbal extracts, sitology-acceptable food supplementary or other natural carbohydrates may be added as an additional ingredient.

The food-aid additive described above is added to produce the health functional food of each formulation and can be appropriately selected and used by a person skilled in the relevant field of technology. For example, various nutrient additives, vitamins, minerals (electrolytes), synthetic flavors and natural flavors, colorants and fillers, pectic acid and its salts, alginic acid and its salts, organic acids, protective colloid thickeners, pH controller, stabilizer, preservative, glycerin, alcohol, carbonating agent used in a carbonated drink, and the like, but the kind is not limited by the above.

In addition, the health functional food described above may contain additional ingredients which are commonly used in food to improve smell, taste, visual appearance and the like. For example, vitamins A, C, D, E, B1, B2, B6, B12, niacin, biotin, folate, panthotenic acid and the like can be included. In addition, it may include minerals such as zinc (Zn), iron (Fe), calcium (Ca), chromium (Cr), magnesium (Mg), manganese (Mn) and copper (Cu) and the like. It may also contain amino acids such as lysine, tryptophan, cysteine, valine and the like.

In addition, the described health functional food may include one or more preservatives (such as potassium sorbate, sodium benzoate, salicylic acid, and sodium dehydroacetate), bactericides (such as bleaching powder and high bleaching powder, sodium hypochlorite), antioxidants (butylhydroxyanilide (BHA), butylhydroxytoluene (BHT), etc.), coloring agents (such as tar pigments), color formers (such as sodium nitrite and sodium acetates), bleaching agents (sodium sulfite), seasonings (such MSG, sodium glutamate), sweeteners (such as dulcin, cyclamate, saccharin, sodium), flavorings (vanillin, lactones, etc.), swelling agents (alum, potassium hydrogen D-tartrate), fortifier, emulsifiers, thickeners, encapsulating agents, gum bases, foam inhibitors, solvent, improver, and the like. The above additives are selected according to the type of food and used in an appropriate amount.

The health functional food composition can be prepared by a method commonly used in the industry and can be prepared by adding raw materials and ingredients which are conventionally added in the industry. In addition, unlike general medicine, the health functional food may have an advantage, fo example, as there can be no side effect from a long-term use and have better portability.

The pharmaceutical composition may be prepared and administered in a wide variety of dosage formulations. Compounds described may be administered orally, rectally, or by injection (e.g. intravenously, intramuscularly, intracutaneously, subcutaneously, intraduodenally, or intraperitoneally).

For preparing pharmaceutical compositions from compounds described herein, pharmaceutically acceptable carriers can be either solid or liquid. Solid form preparations include powders, tablets, pills, capsules, cachets, suppositories, and dispersible granules. A solid carrier may be one or more substance that may also act as diluents, flavoring agents, binders, preservatives, tablet disintegrating agents, or an encapsulating material.

In powders, the carrier may be a finely divided solid in a mixture with the finely divided active component. In tablets, the active component may be mixed with the carrier having the necessary binding properties in suitable proportions and compacted in the shape and size desired.

The powders and tablets preferably contain from 5% to 70% of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized molds, allowed to cool, and thereby to solidify.

Liquid form preparations include solutions, suspensions, and emulsions, for example, water or water/propylene glycol solutions. For parenteral injection, liquid preparations can be formulated in solution in aqueous polyethylene glycol solution.

Aqueous solutions suitable for oral use can be prepared by dissolving the active component in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations that are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The quantity of active component in a unit dose preparation may be varied or adjusted from 0.1 mg to 10000 mg according to the particular application and the potency of the active component. The composition can, if desired, also contain other compatible therapeutic agents.

Some compounds may have limited solubility in water and therefore may require a surfactant or other appropriate co-solvent in the composition. Such co-solvents include: Polysorbate 20, 60, and 80; Pluronic F-68, F-84, and P-103; cyclodextrin; and polyoxyl 35 castor oil. Such co-solvents are typically employed at a level between about 0.01 % and about 2% by weight. Viscosity greater than that of simple aqueous solutions may be desirable to decrease variability in dispensing the formulations, to decrease physical separation of components of a suspension or emulsion of formulation, and/or otherwise to improve the formulation. Such viscosity building agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose, chondroitin sulfate and salts thereof, hyaluronic acid and salts thereof, and combinations of the foregoing. Such agents are typically employed at a level between about 0.01% and about 2% by weight.

The pharmaceutical compositions may additionally include components to provide sustained release and/or comfort. Such components include high molecular weight, anionic mucomimetic polymers, gelling polysaccharides, and finely-divided drug carrier substrates. These components are discussed in greater detail in U.S. Pat. Nos. 4,911,920; 5,403,841; 5,212,162; and 4,861,760.

The pharmaceutical composition may be intended for intravenous use. The pharmaceutically acceptable excipient can include buffers to adjust the pH to a desirable range for intravenous use. Many buffers including salts of inorganic acids such as phosphate, borate, and sulfate are known.

### Effective Dosages

The pharmaceutical composition may include compositions wherein the active ingredient is contained in a therapeutically effective amount, i.e., in an amount effective to achieve its intended purpose. The actual amount effective for a particular application will depend, *inter alia,* on the condition being treated.

The dosage and frequency (single or multiple doses) of compounds administered can vary depending upon a variety of factors, including route of administration; size, age, sex, health, body weight, body mass index, and diet of the recipient; nature and extent of symptoms of the disease being treated; presence of other diseases or other health-related problems; kind of concurrent treatment; and complications from any disease or treatment regimen. Other therapeutic regimens or agents can be used in conjunction with the methods and compounds disclosed herein.

Dosages may be varied depending upon the requirements of the subject and the compound being employed. The dose administered to a subject, in the context of the pharmaceutical compositions presented herein, should be sufficient to effect a beneficial therapeutic response in the subject over time. The size of the dose also will be determined by the existence, nature, and extent of any adverse side effects. Generally, treatment is initiated with smaller dosages, which are less than the optimum dose of the compound. Thereafter, the dosage is increased by small increments until the optimum effect under circumstances is reached.

Dosage amounts and intervals can be adjusted individually to provide levels of the administered compounds effective for the particular clinical indication being treated. This will provide a therapeutic regimen that is commensurate with the severity of the individual's disease state.

Utilizing the teachings provided herein, an effective prophylactic or therapeutic treatment regimen can be planned that does not cause substantial toxicity and yet is entirely effective to treat the clinical symptoms demonstrated by the particular patient. This planning should involve the careful choice of active compound by considering factors such as compound potency, relative bioavailability, patient body weight, presence and severity of adverse side effects, preferred mode of administration, and the toxicity profile of the selected agent.

### Toxicity

The ratio between toxicity and therapeutic effect for a particular compound is its therapeutic index and can be expressed as the ratio between LD₅₀ (the amount of compound lethal in 50% of the population) and ED₅₀ (the amount of compound effective in 50% of the population). Compounds that exhibit high therapeutic indices are preferred. Therapeutic index data obtained from cell culture assays and/or animal studies can be used in formulating a range of dosages for use in humans. The dosage of such compounds preferably lies within a range of plasma concentrations that include the ED₅₀ with little or no toxicity. The dosage may vary within this range depending upon the dosage form employed and the route of administration utilized. See, *e.g.* Fingl et al., In: THE PHARMACOLOGICAL BASIS OF THERAPEUTICS, Ch.1, p.1, 1975. The exact formulation, route of administration, and dosage can be chosen by the individual physician in view of the patient's condition and the particular method in which the compound is used.

When parenteral application is needed or desired, particularly suitable admixtures for the compounds included in the pharmaceutical composition may be injectable, sterile solutions, oily or aqueous solutions, as well as suspensions, emulsions, or implants, including suppositories. In particular, carriers for parenteral administration include aqueous solutions of dextrose, saline, pure water, ethanol, glycerol, propylene glycol, peanut oil, sesame oil, polyoxyethylene-block polymers, and the like. Ampoules are convenient unit dosages. Pharmaceutical admixtures suitable for use in the pharmaceutical compositions presented herein may include those described, for example, in Pharmaceutical Sciences (17th Ed., Mack Pub. Co., Easton, PA) and WO 96/05309, the teachings of both of which are hereby incorporated by reference.

### III. Methods of Treatment

Disclosed in the present application is a method for preventing or treating Acute Radiation Syndrome comprising the step of administering PLAG (1-palmitoyl-2-linoleoyl-3-acetylglycerol) to a subject.

Definition of PLAG (also referred to herein as EC-18) and acute radiation syndrome are described above.

Since PLAG has preventive and therapeutic effects on acute radiation syndrome, it is possible to prevent or treat the acute radiation syndrome by administering a composition containing PLAG to the individual.

PLAG (1-palmitoyl-2-linoleoyl-3-acetylglycerol) has an excellent effect in preventing and treating acute radiation syndrome by increasing the survival rate of subjects with radiation exposure. Accordingly, the pharmaceutical composition and the health functional food composition containing the PLAG as an active ingredient of the present invention can be effectively used for preventing, treating or improving acute radiation syndrome.

### IV. Examples

Although the foregoing section has been described in some detail by way of illustration and example for purposes of clarity of understanding, it is apparent to those skilled in the art that certain minor changes and modifications will be practiced in light of the above teaching. Therefore, the description and examples should not be construed as limiting the scope of any invention described herein.

### Example 1: Effect of PLAG (1-palmitoyl-2-linoleoyl-3-acetylglycerol) on the reduction of immune cell levels in the blood of an animal model of acute radiation syndrome (ARS)

Balb /c mice (9-week-old male) were purchased from Koatech (Pyeongtaek, Republic of Korea) and maintained in a specific pathogen-free (SPF) environment to establish an animal model of acute radiation syndrome. In order to evaluate the effect of PLAG on the reduction of immune cells by radiation, the following three groups were constructed for this study. (1) normal control group (positive control); (2) Radiotherapy-Radiation induced leukopenia (RIL) group (negative control group); And (3) RIL + PLAG treated group (experimental group).

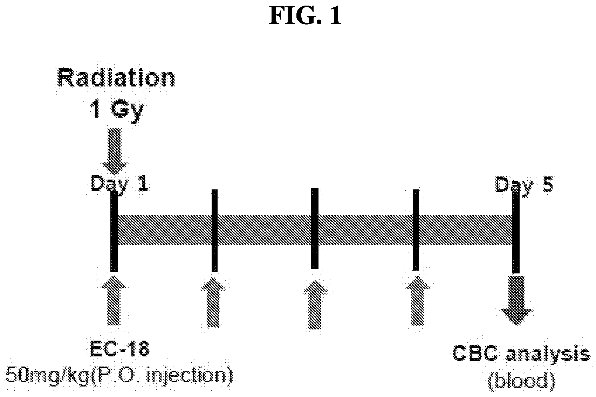

Specifically, mice treated with RIL and RIL + PLGA were exposed to whole body radiation at 1 Gy (100 rad = 1.06 min). For the RIL + PLAG treatment group, 50 mg / kg / day of PLAG (Enzychem Lifesciences Co., Daejeon, Republic of Korea) was orally administered daily for 4 consecutive days after irradiation. The normal controls were not exposed to radiation. On the 5th day after irradiation, blood samples were collected, and then Complete Blood Count (CBC) was measured using a Mindray BC-5300 auto hematology analyzer (Shenzhen Mindray Bio-medical Electronics, China) so that the number of blood leukocytes, neutrophils and lymphocytes in blood could be counted (FIG. 1).

As a result, in the RIL + PLAG treated group, the blood level of leukocyte, neutrophil and lymphocyte counts were 63% (2.18 ± 0.31 vs 3.56 ± 0.84), 34% (0.87 ± 0.04 vs. 1.17 ± 0.35) and 85% ± 0.25 vs. 2.2 ± 0.42) (Table 1 and FIG. 2) increased in statistically significant manners.

**Table 1**

| | Normal Control (n=3) | RIL (n=3) | RIL + PLAG (n=3) |
|---|---|---|---|
| White blood cells (10³/µL) | 7.76 ± 1.96 | 2.18 ± 0.31 | 3.56 ± 0.84 |
| Neutrophils (10³/µL) | 1.32 ± 0.53 | 0.87 ± 0.04 | 1.17 ± 0.35 |
| Lymphocyte (10³/µL) | 6.05 ± 1.36 | 1.19 ± 0.25 | 2.2 ± 0.42 |

Therefore, it can be seen from the above results that PLAG exhibits an effect of inducing an increase of various immune cells levels which were reduced by radiation treatment in the ARS study model.

### Example 2: The Effect of PLAG on Survival Rate in Acute Radiation Syndrome (ARS) Animal Model

In order to further confirm the effect of PLAG on the acute radiation syndrome, an animal model of radiation-induced oral mucositis was prepared and tested as an acute radiation syndrome animal model with severer conditions than those in Example 1 above. First, Balb / c mice (8-week-old female) were purchased from Koatech (Republic of Korea) and maintained in a specific pathogen-free (SPF) environment to establish a radiation-induced oral mucositis model. In this example, first group of oral mucositis induced group (negative control) was compared to the second group where PLAG was orally administered to animals with induced oral mucositis.

Specifically, mice were exposed to 1 Gy whole body gamma radiation and scars of 0.2 cm on the mice tongue were scratched at 0, 7, 10 and 16 days with the same force and depth using an 18 gauge needle. On day 2 post-irradiation, 5-Fluorouracil (50 mg / kg / day) was intraperitoneally administered and PLAG was administered orally at 250 mg / kg / day for 18 days (Table 2).

**Table 2**

| | OM induced group | OM induced + PLGA administered group |
|---|---|---|
| Number of subjects | 7 (female) | 7 (female) |
| Scratch on tongue (0, 2, 10 and 16 days) | 0.2 cm | 0.2 cm |
| Gamma ray (day 2) | 1 Gy | 1 Gy |
| dosage/day | PBS | PLAG (250 mg/kg) |
| Method of administration/days | Oral 0 or 18 days | Oral 0 or 18 days |
| CBC analysis/schedule | 7 and 10 days | 7 and 10 days |

As a result, in the oral mucositis-induced group, the survival rate was only 28% (2/7, 72%) at day 18, whereas 85% (6/7, 15% (FIG. 3) survival rate was reported from OM-induced + PLAG administration Group. These results suggest that PLAG treatment significantly increased the survival rate of mice despite with the reduction of hematopoietic cells from the gamma ray exposure and 5-Fluorouracil treatment and the increased risk of infection caused by oral wounds with scratch on tongue. It further suggests that PLAG exhibits excellent prophylactic and therapeutic effects.

### Example 3: Effect of PLAG on each symptom affecting survival rate in an animal model of acute radiation syndrome

For the animal model used in Example 2 above, oral mucositis level and neutrophil count in blood were determined.

Oral mucositis level was determined by counting the ulcer formation and edema of the tongue and the incidence of wound atrophy, and calculating the score of oral mucositis by a total of 5 blinded observers. On the 7th and 10th day, the blood of the experimental groups was collected and the number of neutrophils in the blood was measured by measuring the Complete Blood Count (CBC) using a Mindray BC-5300 auto hematology analyzer (Shenzhen Mindray Bio-medical Electronics, China).

As a result, visually confirmed oral mucositis levels were significantly reduced in the PLAG-treated group compared to the non-PLAG-treated group with less ulcer formation and edema of the tongue and the lower incidence of wound atrophy. (FIG. 4) The oral mucositis score was also significantly decreased (FIG. 5) On the 7th and 10th days, neutrophil counts in the blood were decreased by approximately 95% in the blood from radiation-induced oral mucositis animal group at 10 days, but neutrophil number was significantly increased in the group administered with PLAG daily (FIG. 6).

### Example 4: Improvement of Survival Rate

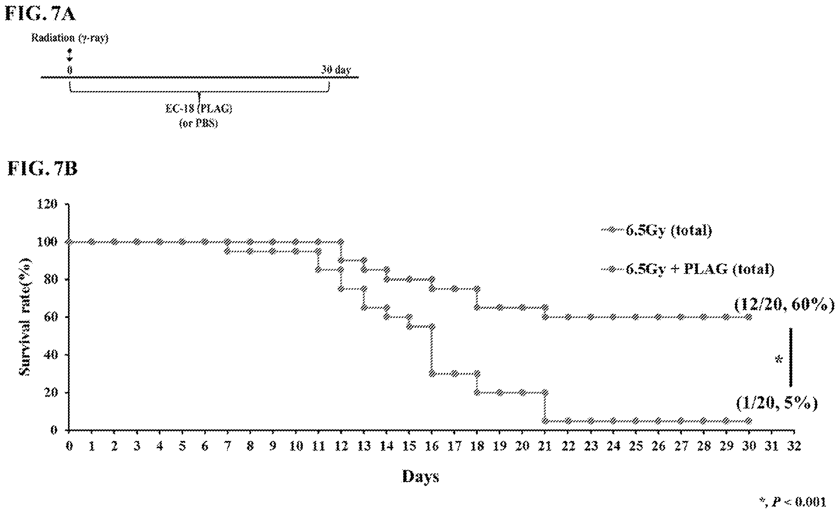

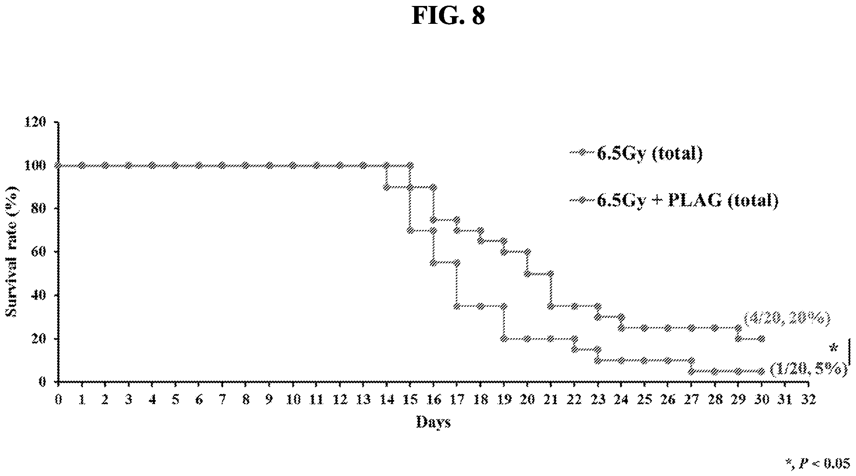

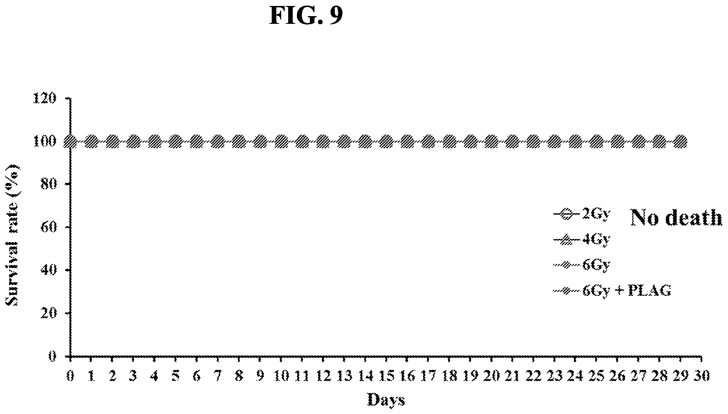

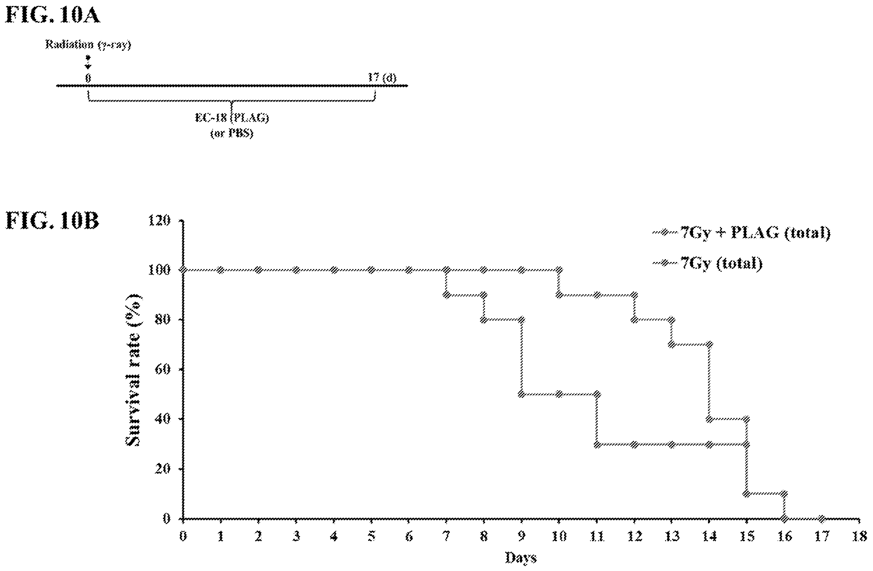

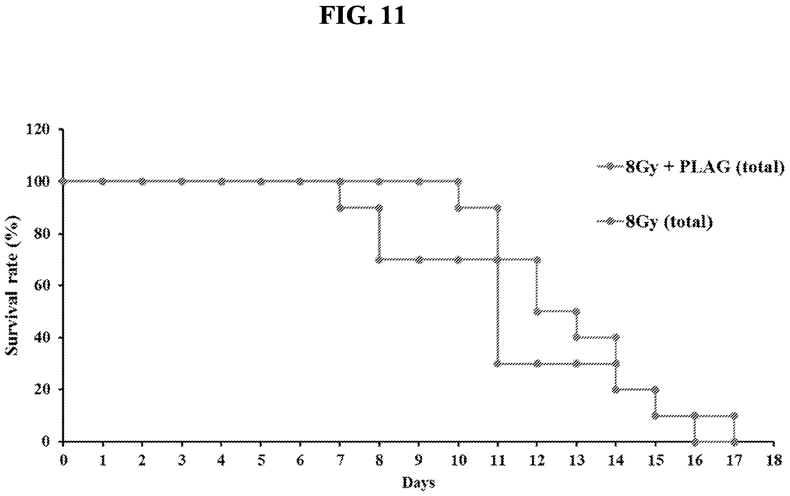

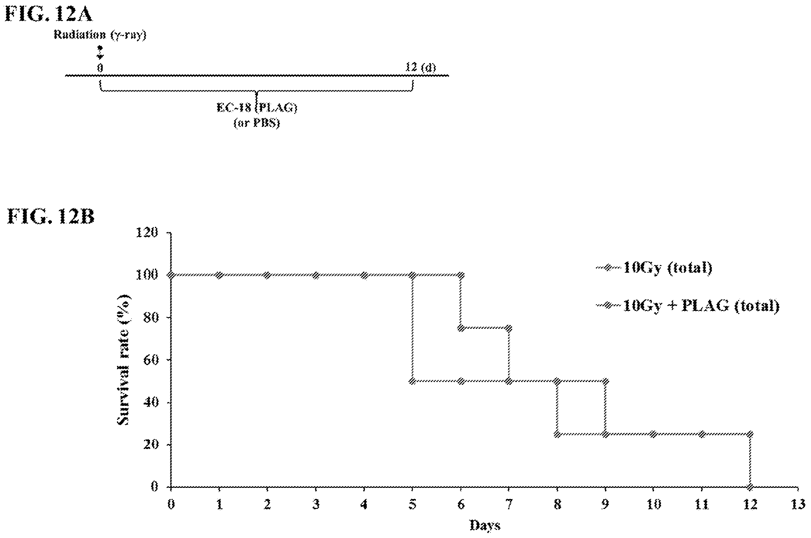

In order to evaluate effects of PLAG on survival rates of animal models exposed to radiation, the following test groups were constructed as shown in Table 3 and results are shown in FIG. 7B, and FIG. 8-12. As discussed in Examples above, the first group of untreated group (negative control) of ARS-induced mice after radiation was compared to the second group of treated group of ARS-induced mice with PLAG after radiation.

**Table 3**

| | Test Groups | | | | |
|---|---|---|---|---|---|
| FIG. 7B | • Balb/c : 11 weeks, male (n=10) or Female (n=10) | | | | |
| | • γ-Radiation : 6.5Gy (100 rad=1.06min), TBI | | | | |
| | • EC-18 : 250 mg/kg/day (P.O.) | | | | |
| | • Group: | | | 1) untreated group after radiation | |
| | | | | 2) EC-18 group treated group after radiation | |
| FIG. 8 | • Balb/c : 9 weeks, male (n=10) or Female (n=10) | | | | |
| | • γ-Radiation : 6.5Gy (100 rad=1.06min), TBI | | | | |
| | • EC-18 : 250 mg/kg/day (P.O.) | | | | |
| | • Group: | | | 1) untreated group after radiation | |
| | | | | 2) EC-18 group treated group after radiation | |
| FIG. 9 | • Balb/c : 9 weeks, male (n=5) or Female (n=5) | | | | |
| | • γ-Radiation : 2, 4, 6 Gy (100 rad=1.06min), TBI | | | | |
| | • EC-18 : 250 mg/kg/day (P.O.) | | | | |
| | • Group: | | | 1) untreated group after radiation (2Gy) | |
| | | | | 2) untreated group after radiation (4Gy) | |
| | | | | 3) untreated group after radiation (6Gy) | |
| | | | | 4) EC-18 group treated group after radiation (6Gy) | |
| FIG. 10B | • Balb/c : 9 weeks, male (n=5) or Female (n=5) | | | | |
| | • γ-Radiation :7 Gy (100 rad=1.06min), TBI | | | | |
| | • EC-18 : 250 mg/kg/day (P.O.) | | | | |
| | • Group: | | | | 1) untreated group after radiation |
| | | | | | 2) EC-18 group treated group after radiation |
| FIG. 11 | • Balb/c : 9 weeks, male (n=5) or Female (n=5) | | | | |
| | • γ-Radiation : 8 Gy (100 rad=1.06min), TBI | | | | |
| | • EC-18 : 250 mg/kg/day (P.O.) | | | | |
| | • Group: | | | 1) untreated group after radiation | |
| | | | | 2) EC-18 group treated group after radiation | |
| FIG. 12 | • Balb/c : 9 weeks, male (n=2) or Female (n=2) | | | | |
| | • γ-Radiation : 10 Gy (100 rad=1.06min), TBI | | | | |
| | • EC-18 : 250 mg/kg/day (P.O.) | | | | |
| | | • Group: | | | 1) untreated group after radiation |
| | | | | | 2) EC-18 group treated group after radiation |

### Example 5: Treatment of Erythema

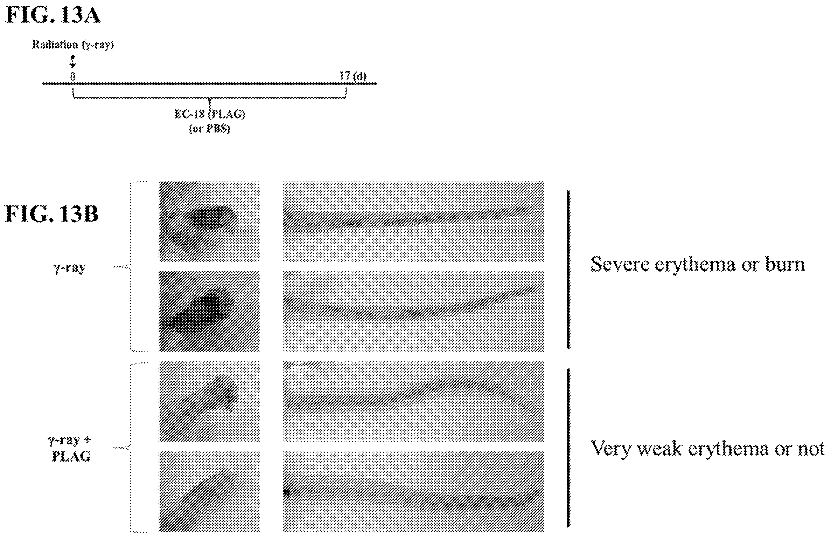

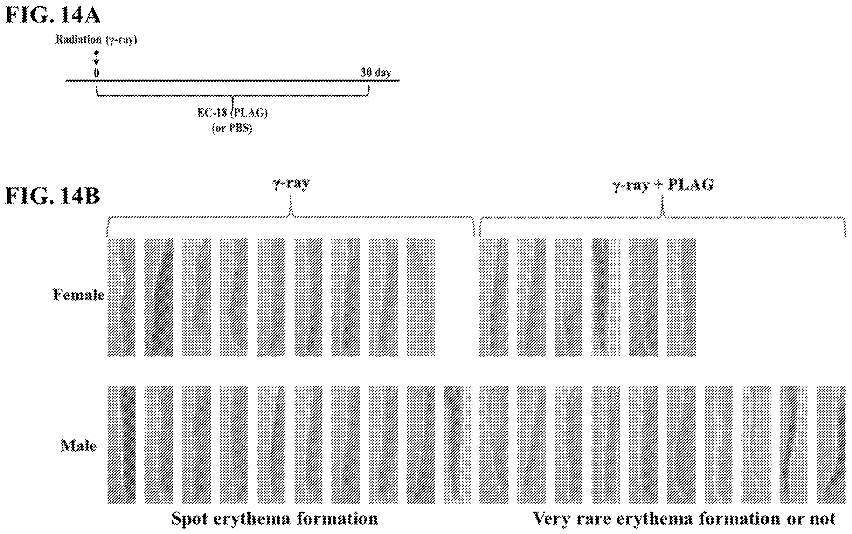

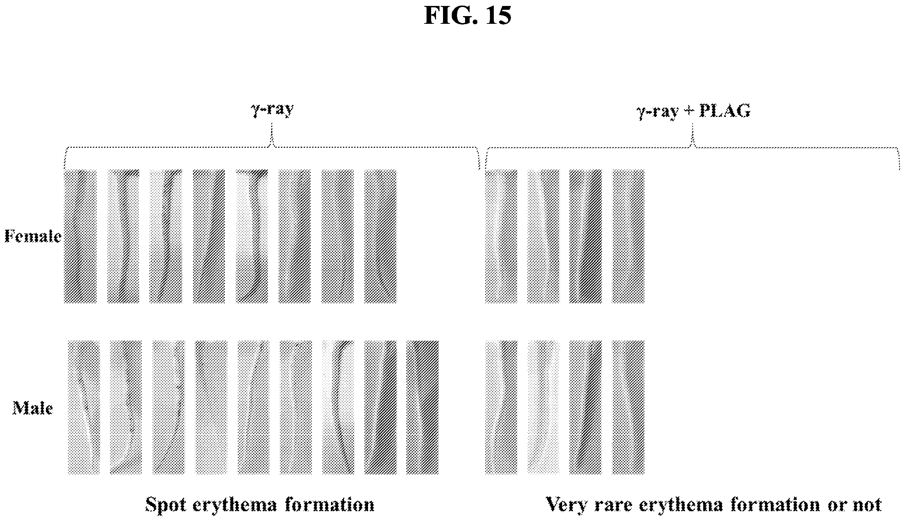

In order to evaluate effects of PLAG on recovery skin damage of animal models exposed to radiation, the following test groups were constructed as shown in Table 4 and results are shown in FIGS. 13-15. As discussed in Examples above, the first group of untreate group (negative control) of mice after radiation was compared to the second group of treated group of mice with PLAG after radiation.

**Table 4**

| | Experiment Group | |
|---|---|---|
| FIG. 13B | • Balb/c : 9 weeks, male (n=5) or Female (n=5) | |
| | • γ-Radiation : 8 Gy (100 rad=1.06min), TBI | |
| | • EC-18 : 250 mg/kg/day (P.O.) | |
| | • Group: | 1) untreated group after radiation |
| | | 2) EC-18 group treated group after radiation |
| FIG. 14B | • Balb/c : 9 weeks, male (n=10) or Female (n=10) | |
| | • γ-Radiation : 6.5Gy (100 rad=1.06min), TBI | |
| | • EC-18 : 250 mg/kg/day (P.O.) | |
| | • Group: | 1) untreated group after radiation |
| | | 2) EC-18 group treated group after radiation |
| FIG. 15 | • Balb/c : 11 weeks, male (n=10) or Female (n=10) | |
| | • γ-Radiation : 6.5 Gy (100 rad=1.06min), TBI | |
| | • EC-18 : 250 mg/kg/day (P.O.) | |
| | • Group: | 1) untreated group after radiation |
| | | 2) EC-18 group treated group after radiation |

### Example 6: Effect on anti-apotosis of HaCaT cells

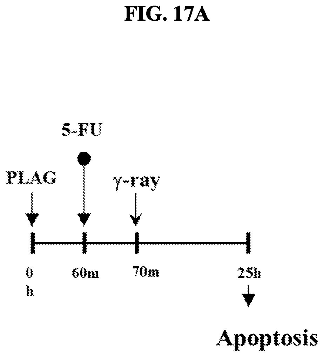

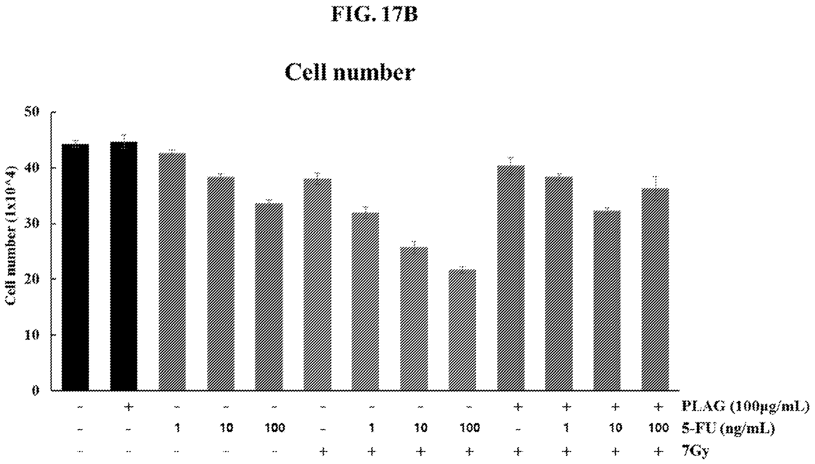

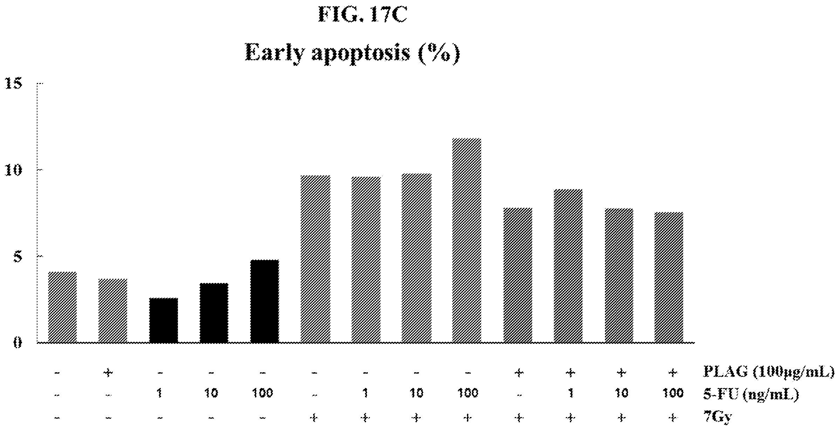

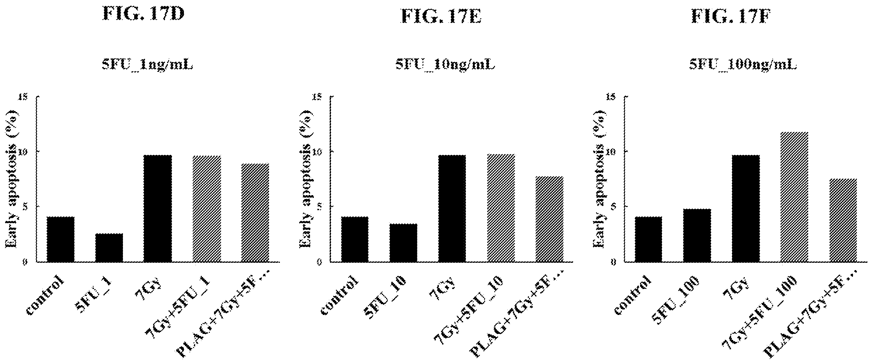

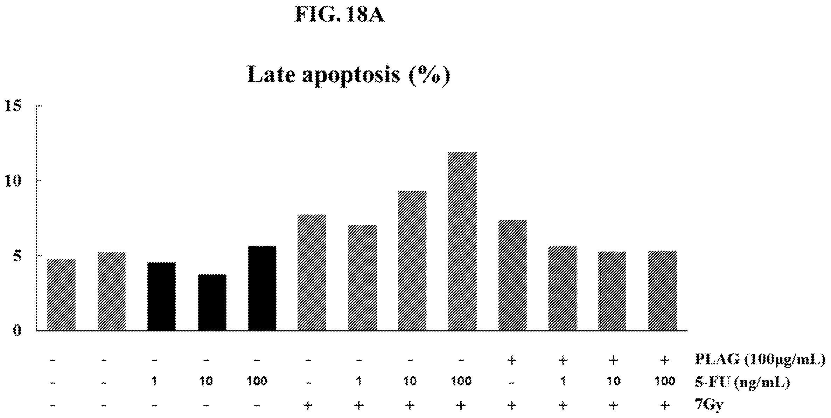

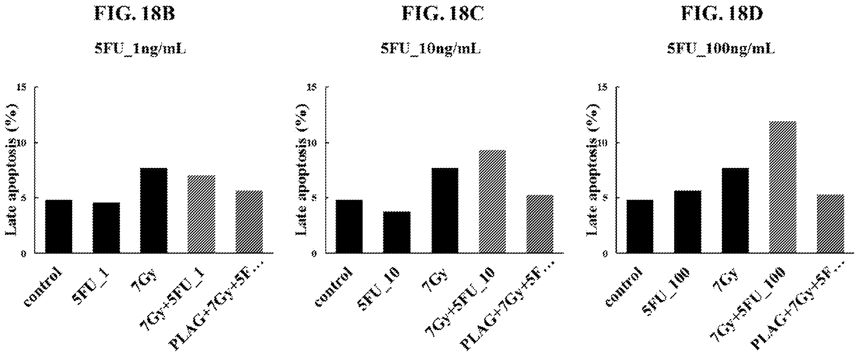

In order to evaluate effects of PLAG on HaCaT cells exposed to radiation, test groups were constructed as follows and results are shown in FIGS. 17B-18D.
- HaCaT : human keratinocyte
- PLAG : 100µg/mL for 1hr pretreatment
- 5-FU : 1, 10, 100 ng/mL
- Gamma radiation : 7 Gy
- apoptosis check after 24 hours
- Cell counting after Trypan blue staining
- Apoptosis (Annexin V + 7AAD)

### Example 7: Effect on Reactive Oxygen Species (ROS) of HaCaT cells

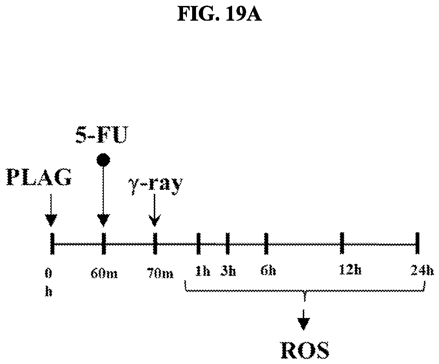

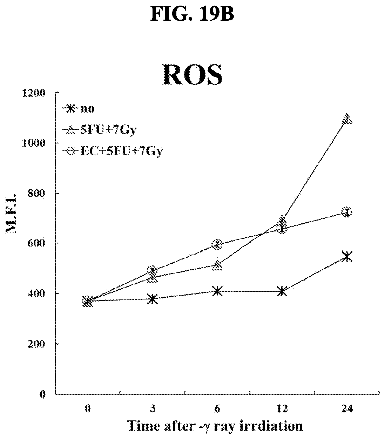

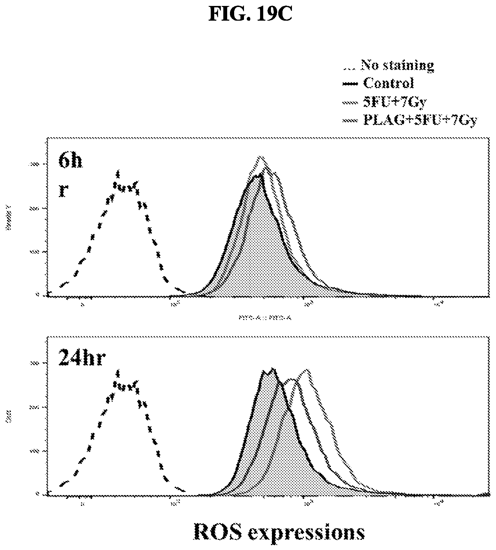

In order to evaluate effects of PLAG on HaCaT cells exposed to radiation, test groups were constructed as in Example 6 and results are shown in FIGS. 19A-19C.

### Example 8: Improvement of Survival Rate

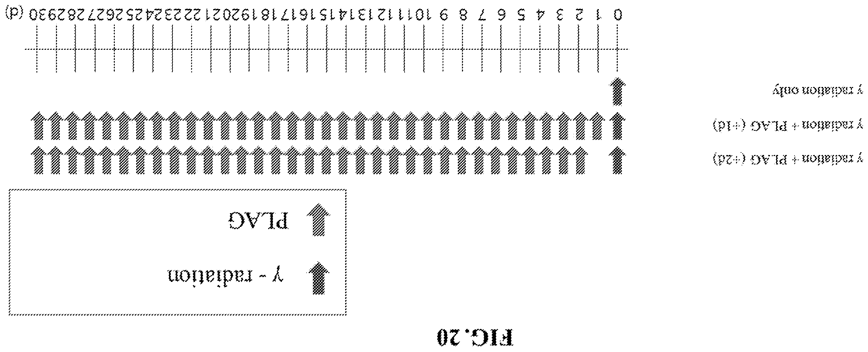

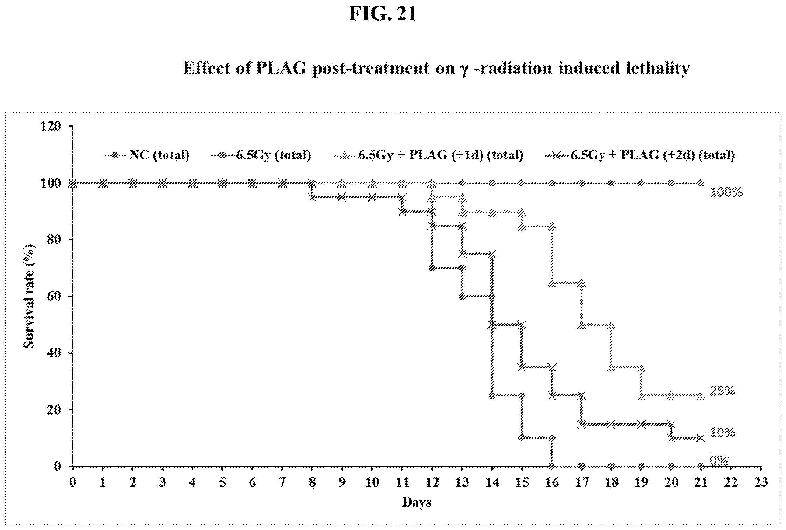

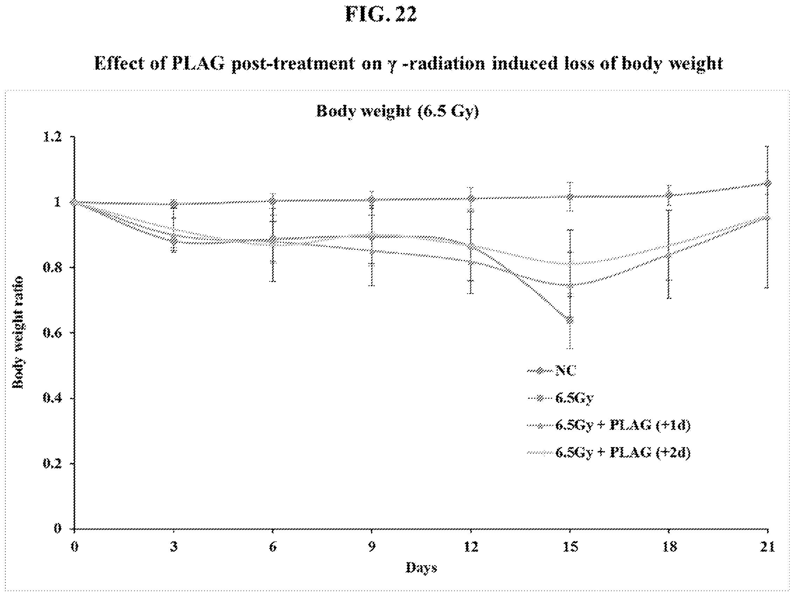

FIG. 20 is an exemplary diagram for testing survival rates of treatment on 6.5 Gy of γ-radiation induced Acute Radiation Syndrome animal model (mouse, BALB/c, 11 weeks). FIG. 21 shows the results of survival curves for Balb/c mice exposed to 6.5 Gy of γ-radiation in the daily treatment of EC-18 after irradiation. NC represents untreated control without radiation; 6.5Gy is the survival rate of the mouse radiated with 6.5 Gy of γ-radiation; 6.5Gy + PLAG (+1d) is the survival rate of the mouse radiated with 6.5 Gy of γ-radiation and treated with PLAG for 1 day; and 6.5Gy + PLAG (+2d) is the survival rate of the mouse radiated with 6.5 Gy of γ-radiation and treated with PLAG for 2 day. FIG. 22 shows changes in body weights of Balb/c mice in FIG. 21.

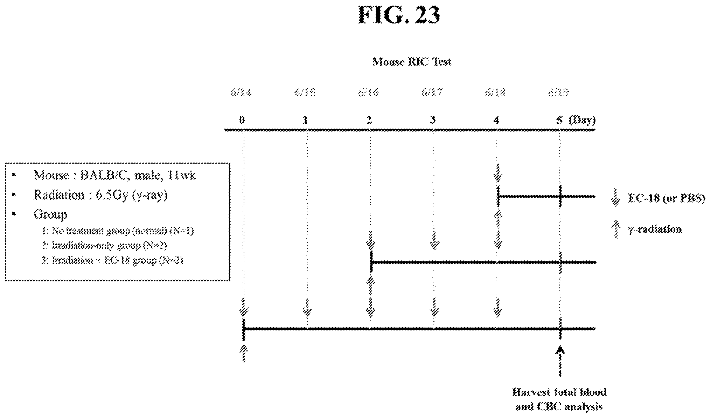

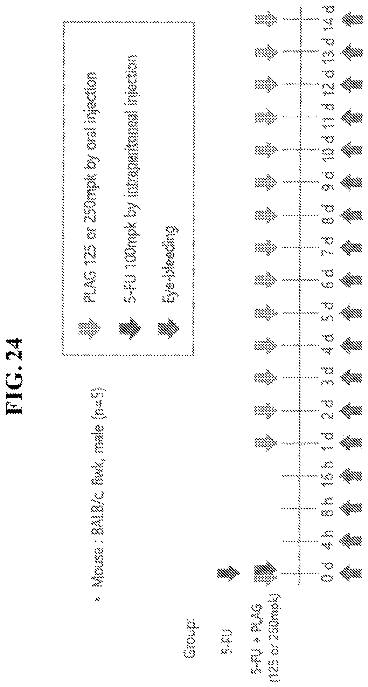

Various tests to measure survival rates after γ-radiation to animal models can be set. For instance, FIG. 23 depicts an exemplary diagram for an exemplary experiment to test survival rate in the mouse models. FIG. 24 depicts an exemplary diagram for an exemplary experiment including three groups (5-FU only, 5-FU + PLAG 125 mg/kg, 5-FU + PLAG 250 mg/kg) to check time kinetics of neutrophil in 5-FU (100 mg/kg) induced neutropenia.

### Example 9: Effects of PLAG measured in vitro (western blot)

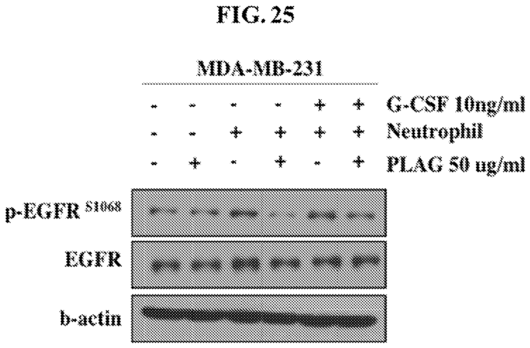

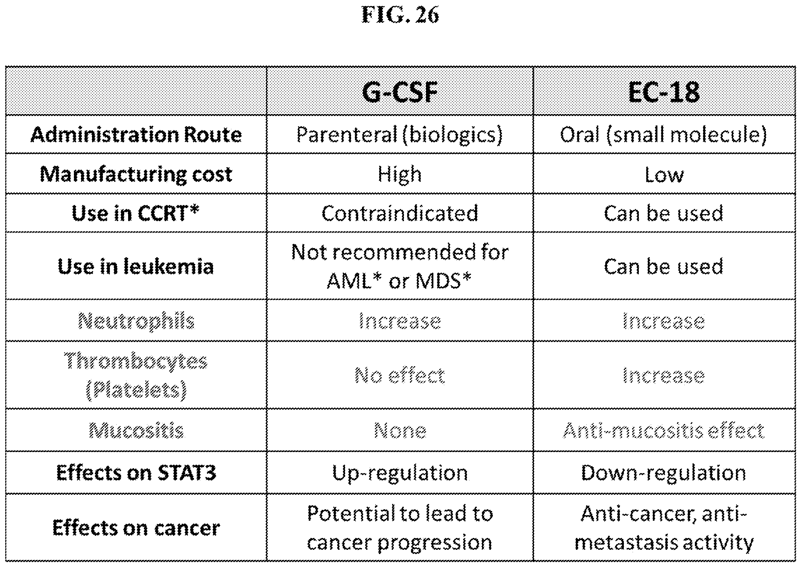

The effects of EC-18 in comparison to G-CSF to cells after γ-radiation were measured by western blot (FIG. 25). The experiment can i) show correlation between EGFR activity and the abnormal growth and metastasis induction of breast cancer cells in the TAN environment and ii) confirm inhibitory effect mechanism by PLAG treatment in this condition. In fact, it was confirmed that EGFR activity was increased in the groups stimulated with neutrophil or G-CSF-activated neutrophils, however, the activity of EGFR (Phosphorylation of EGFR) was decreased in the group treated with PLAG. FIG. 26 can show effects of PLAG in comparison to G-CSF.

### Example 10: Survival Time of various radiation syndrome

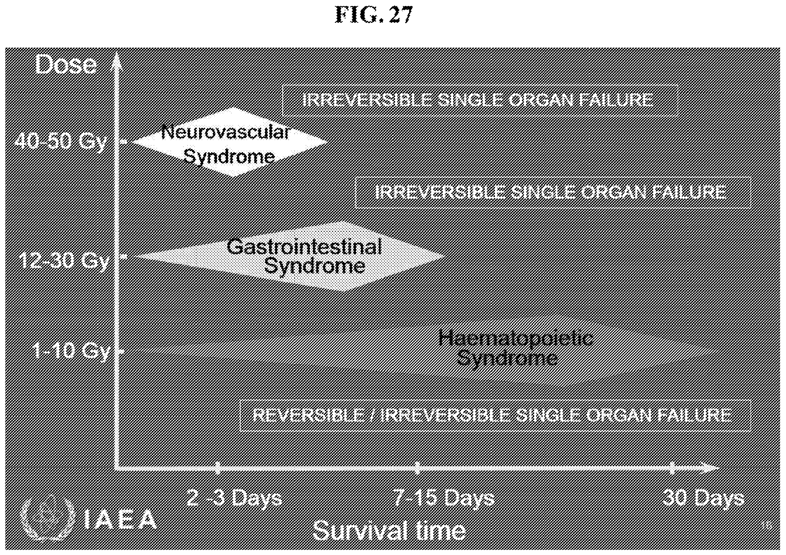

FIG. 27 depicts a diagram of survival time of patients exposed to radiation and various ARSs.

### Example 11: EC-18 for the treatment of Acute Radiation Syndrome

We investigated the efficacy of EC-18 for the development of a medical countermeasure for Acute Radiation Syndrome (ARS) by analyzing IR-induced mortality and morbidity.

### MATERIALS AND METHODS

### Animals

Specific-pathogen-free male and female BALB/c mice (10 weeks of age) were obtained from Koatech Co. (Pyongtaek, Republic of Korea). Upon receipt, the mice were housed, 5 per cage, in a specific pathogen-free facility and acclimatized for 1 week under conditions of consistent temperature and normal light cycles. All the animals were fed a standard mouse diet with water allowed ad libitum. All experimental procedures were approved by the Institutional Animal Care and Use Committee of the Korea Research Institute of Bioscience and Biotechnology and were performed in compliance with the National Institutes of Health guidelines for the care and use of laboratory animals and Korean national laws for animal welfare.

### Determination of lethality dose (LD) of total body γ-radiation (TBI)

Whole-body irradiation of the animals was carried out with Gamma Irradiator (J.L. Shepherd & Associates, San Fernando, USA) with a ⁶⁰Co source (exposure rate 0.833Gy/min). For the experiments to determine survival rate following γ-irradiation, mice were grouped 20 (10 males and 10 females) per treatment cohorts; 6.0Gy-exposed cohort, 6.2Gy-exposed cohort, 6.4Gy-exposed cohort and 6.5Gy-exposed cohort. The survival and body weight of the animals was recorded daily for 30 days.

### Establishment of a murine model of γ-radiation-induced acute radiation syndrome (ARS)

For the experiments to determine survival rate following LD70/30 dose 6.11 Gy of γ-irradiation (TBI, ⁶⁰Co, 0.833Gy min-1), mice were grouped 20 (10 males and 10 females) per treatment cohorts; γ-radiation only cohort (positive control), γ-radiation with EC-18 10 mg/kg cohort, γ-radiation with EC-18 50 mg/kg cohort, and γ-radiation with EC-18 250 mg/kg cohort. EC-18 (Enzychem Lifesciences, Jaecheon, Republic of Korea) was suspended in PBS and orally administrated once a day, starting 1 day after irradiation. The positive control group was administrated PBS. The survival and body weight of the animals were recorded daily for 30 days.

For the time course analysis of hematopoietic ARS (H-ARS), female mice were grouped 5 per treatment cohorts; γ-radiation only cohort (positive control) and γ-radiation with EC-18 250 mg/kg cohort. For the analysis of dose effect of EC-18 administration on H-ARS, mice were grouped 8 (5 males and 3 females) per treatment cohorts; γ-radiation only cohort (positive control), γ-radiation with EC-18 50 mg/kg cohort, γ-radiation with EC-18 100 mg/kg cohort, γ-radiation with EC-18 250 mg/kg cohort and γ-radiation with EC-18 100 mg/kg cohort. The whole blood was collected from the orbital sinuses using EDTA-free capillary tubes (Kimble Chase Life Science and Research Products LLC, FL, USA) and collection tubes containing K3E-K3EDTA (Greiner Bio-One International, Kremsmünster, Austria). The blood cells were counted and classified by complete blood count (CBC) analysis using Mindray BC-5000 auto-hematology analyzer (Shenzhen Mindray Biomedical Electronics, Guangdong Sheng, China). The values of the blood cells of the animals were recorded daily for 30 days.

### Statistical analyses

All data are presented as the mean ± standard deviation (SD). A paired Log rank (Mantel-Cox) test of survival curve was applied to estimate the significance on survival rate and to compare the duration of neutropenia between control and EC-18-treated cohorts. Average life span was calculated as the sum of the life span for all mice/ total number of mice. For comparison of the statistical differences of more than two groups, one-way ANOVA test was used. All other statistical analyses were performed using a Student's paired t test and p values < 0.05 were considered statistically significant.

### RESULTS

### Radiation dose-response relationship (DRR) and LDXX/30

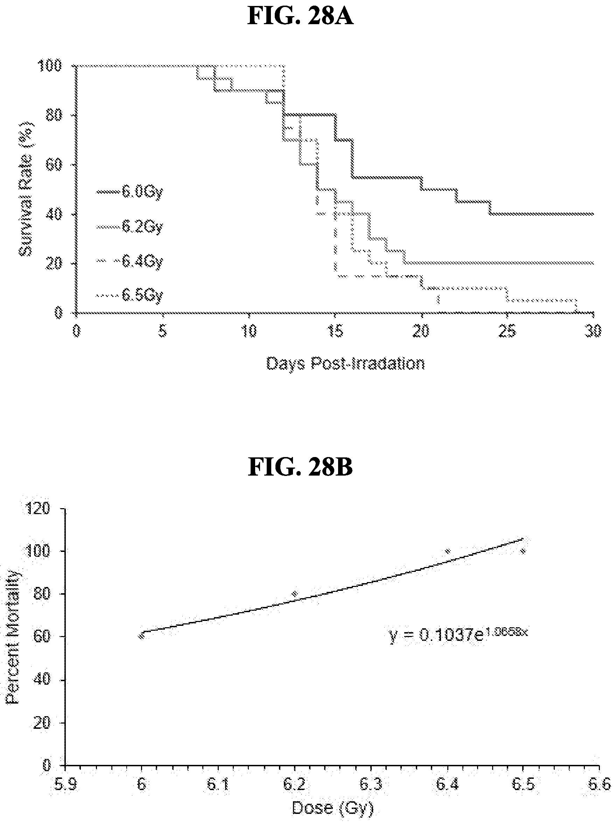

Radiation dose is a significant predictor of mortality and morbidity with increasing doses. FIG. 28A shows the survival rate of BALB/c mice exposed to various doses of ⁶⁰Co γ-radiation. The dose of 6.0, 6.2, 6.4 and 6.5 Gy led to 60, 80, 100 and 100% mortality, respectively, after 30 days of observation. FIG. 28B shows the radiation dose relationship (DRR) using probit models. The mean survival time of decedents for each radiation dose cohort was 15.30 ± 4.98, 13.69 ± 3.26, 14.15 ± 3.48 and 15.85 ± 4.42 days, respectively (Table 5). Thirty-day survival was calculated at each radiation dose and is shown as percent mortality on the Y-axis. Based on the probit model, we determined the LD30/30, LD50/30, LD70/30, and LD95/30 with 95% confidence intervals around each dose (Table 6). The LD70/30 was determined to be 6.11 Gy, and was used to evaluate several biological indicators (e.g. survivability, body weight reduction, and reduction of hematopoietic cells) in subsequent studies.

| **Table 5. 30-Day Mortality of BALB/c Mice After γ-Radiation** | | | |
|---|---|---|---|
| Radiation dose (Gy) | Mortality | Survival time of decedents (days) | |
| | | Mean ± SD | Median |
| 6.00 | 12/20 (60%) | 15.30 ± 4.98 | 15.50 |
| 6.20 | 16/20 (80%) | 13.69 ± 3.26 | 13.50 |
| 6.40 | 20/20 (100%) | 14.15 ± 3.48 | 14.00 |
| 6.50 | 20/20 (100%) | 15.85 ± 4.42 | 14.50 |

| **Table 6. Estimated Radiation Dose in BALB/c mice After γ-Radiation** | | | |
|---|---|---|---|
| LD XX/30 | LD estimate (Gy) | Lower 95% CI (Gy) | Upper 95% CI (Gy) |
| LD30/30 | 5.31 | 4.98 | 5.56 |
| LD50/30 | 5.79 | 5.59 | 5.96 |
| LD70/30 | 6.11 | 5.98 | 6.22 |
| LD95/30 | 6.39 | 6.30 | 6.48 |

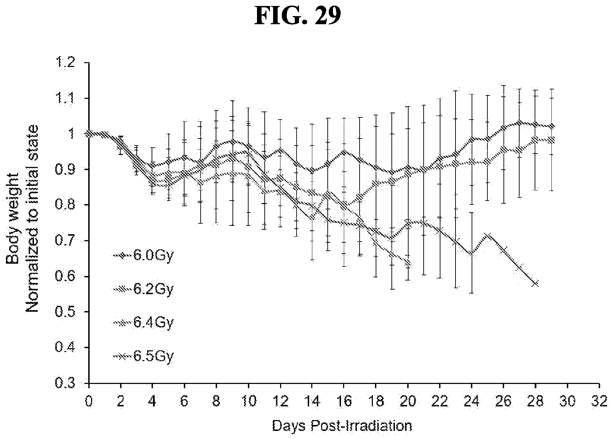

The γ-radiation also caused a substantial reduction of body weight of the irradiated mice (FIG. 29). It was difficult to find a strict relationship between radiation dose and the loss of body weights in the initial stage (0-10 day), but the body weights of lower dose cohorts (6.0 and 6.2Gy) were restored to the normal range at the end of the observation period.

### Therapeutic effect of EC-18 administration on survivability, average life span and body weights of irradiated mice with LD70/30 of TBI

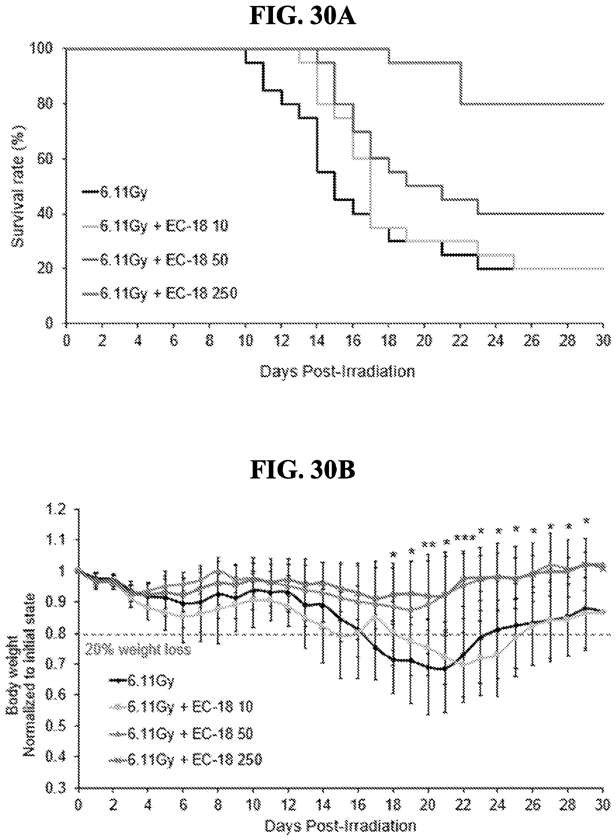

We next investigated the therapeutic effect of EC-18 on survivability of irradiated mice with LD70/30 (6.11 Gy) of TBI over 30 days of the observation period. The survival rate of the positive control cohort was 20% while that of EC-18 10, 50 and 250 mg/kg-treated cohorts was 20, 40 and 80%, respectively (FIG. 30A). Moreover, EC-18 significantly increased the average life span of irradiated mice in a dose-dependent manner (Table 7). The respective average life span of EC-18 10, 50 and 250 mg/kg-treated cohorts was 19.3, 22.3 and 28.2 days as compared to 17.9 days of the positive control cohort.

| **Table 7. Dose-effect of EC-18 administration on survivability and average life duration of irradiated mice.** | | | | | |
|---|---|---|---|---|---|
| | No. of mice that survived/total | Survivability | Average life span | Median survival, days | Log-rank test *p** |
| **Control** | 16/20 | 80% | 28.2 | 30 | <0.0001 |
| **EC-1810 mg/kg** | 8/20 | 40% | 22.3 | 20 | 0.0464 |
| **EC-18 50 mg/kg** | 4/20 | 20% | 19.3 | 17 | 0.4425 |
| **EC-18 250 mg/kg** | 4/20 | 20% | 17.9 | 15 | |

The administration of EC-18 also effectively prevented γ-radiation-induced severe weight loss (FIG. 30B). In particular, EC-18 250 mg/kg-tread cohort significantly reduced γ-radiation-induced weight loss from the 18th day after irradiation to the end of the experiment as compared to the positive control cohort. In addition, the number of mice experiencing a 20% body weight loss from the baseline value sharply decreased as the dose of EC-18 increased (Table 8).

| **Table 8. Dose-effect of EC-18 administration on body weight loss in irradiated mice.** | | | | |
|---|---|---|---|---|
| | ≥10% Body Weight Loss | | ≥20% Body Weight Loss | |
| | n | % | n | % |
| **Control** | 16 | 80 | 8 | 40 |
| **EC-1810 mg/kg** | 17 | 85 | 14 | 70 |
| **EC-18 50 mg/kg** | 11 | 55 | 7 | 35 |
| **EC-18 250 mg/kg** | 3 | 15 | 3 | 15 |

Based on the observations in this study, we concluded that EC-18 has therapeutic potential for improving survivability and preventing body weight loss in γ- radiation-induced ARS.

### Therapeutic effect of EC-18 administration on γ-radiation-induced H-ARS

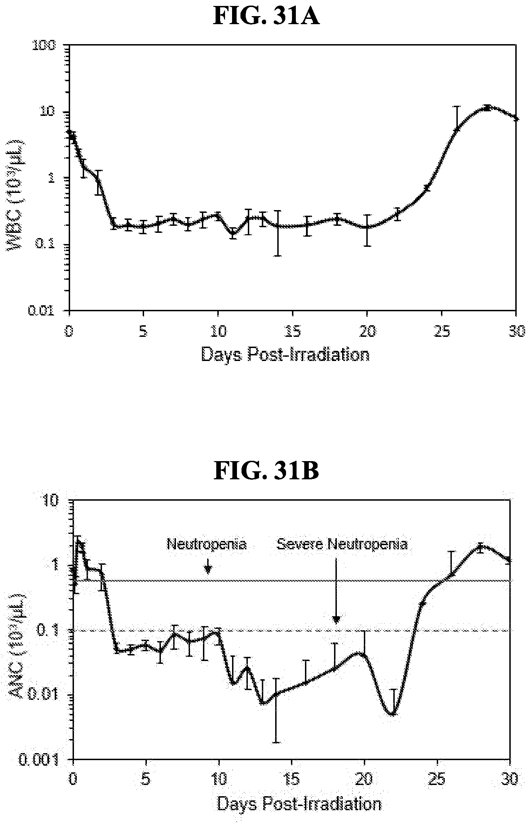

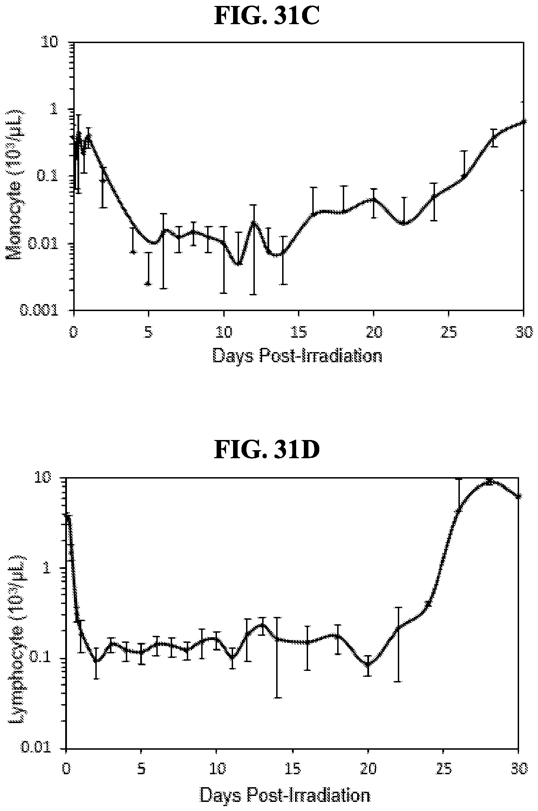

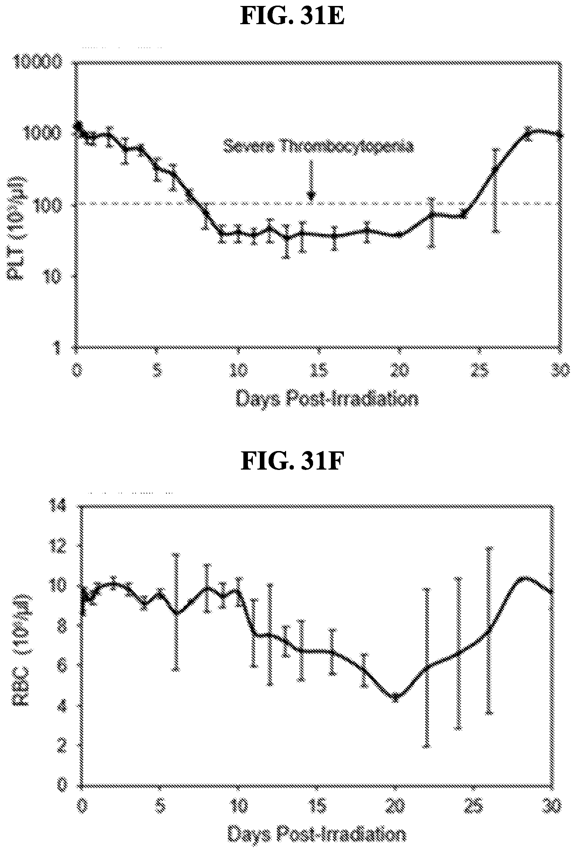

A single TBI of γ-radiation (6.11Gy) rapidly exhausted all kinds of hematopoietic cells including the absolute neutrophil counts (ANC), monocytes, absolute lymphocyte count (ALC), platelet counts (PLT) and red blood cell counts (RBC) within 3days after irradiation (FIG. 31). The mean first day of neutropenia was 2.8±0.45 day, and the duration of neutropenic state was 18.0±1.41 days. All individuals in the irradiated cohort experienced severe neutropenic state (ANC <100 cells/ µL), and the duration of severe neutropenic state was 16±0.00. The mean nadir of ANC after γ-ray irradiation was 0.0±0.00 cells/ µL, and the time of recovery to an ANC≥500 or 1000 cells/ µL was both 27±1.41 days. Moreover, γ-radiation induced greater than 90% reduction in PLT within 7 days after irradiation, and started to recover at 26 days after irradiation. RBC gradually decreased and started to recover at 22 days after irradiation.

The therapeutic effect of EC-18 administration on H-ARS was then evaluated. The administration of EC-18 significantly attenuated γ-radiation-induced depletion of the white blood cell counts (WBC), absolute neutrophil counts (ANC) and absolute lymphocyte counts (ALC) in the irradiated mice (FIGS. 32A-J). Groups of BALB/c mice (n=5, female for FIG. 32A through FIG. 32E and n=8, 5 male, 3 female for FIG. 32F through FIG. 32J) were exposed to 6.11Gy of γ-radiation and various doses of EC-18 were orally administered once daily to day 15, or remained untreated. FIGS. 32A through 32E show the time course of the white blood cell count (WBC; FIG. 32A), absolute Neutrophil count (ANC; FIG. 32B), absolute Lymphocyte count (ALC; FIG. 32C), platelet count (PLT; FIG. 32D) and red blood cell count (RBC; FIG. 32E) over 15 days, respectively. FIGS. 32F through FIG. 32J show the dose effect of EC-18 administration on WBC (FIG. 32F), ANC (FIG. 32G), ALC (FIG. 32H), PLT (FIG. 32I) and BRC (FIG. 32J) on day 15, respectively. ns; not significant, * p<0.05, **p<0.01, *** p<0.005.

The administration of EC-18 substantially reduced γ-radiation-induced reduction of ANC. The mean first day of neutropenia (ANC<500cells/µL) of control and EC-18-treated cohorts was 1.8±1.09 and 2.2±1.09 days (two-sided P value = 0.62), respectively. Although EC-18 did not protect the irradiated mice from experiencing severe neutropenia, it effectively reduced the duration of severe neutropenia from 13.0 days to 7.2±1.79 days. In addition, EC-18 significantly increased the mean nadir of ANC after γ-ray irradiation from 4.0±5.48 cells/µL to 20.0±10.00 cells/µL (two-sided P value = 0.035). The administration of EC-18 attenuated the reduction of PLT and RBC induced by a single TBI (FIGS. 32D, 32E and FIGS. 32I, 32J). Based on the observations, we concluded that EC-18 may have therapeutic potential for attenuating the reduction of blood cells in γ- radiation-induced ARS.

It is understood that the examples and embodiments described herein are for illustrative purposes only and that various modifications or changes in light thereof will be suggested to persons skilled in the art and are to be included within the purview of this application and scope of the appended claims.

## Claims

1. 1-palmitoyl-2-linoleoyl-3-acetylglycerol (PLAG) for use in treating or preventing acute radiation syndrome in a subject.

2. 1-palmitoyl-2-linoleoyl-3-acetylglycerol (PLAG) for use in treating a subject exposed to adverse ionizing radiation.

3. 1-palmitoyl-2-linoleoyl-3-acetylglycerol (PLAG) for use according to claim 1 or 2, wherein the subject is suffering from or susceptible to hematopoietic acute radiation syndrome, radiation-induced coagulopathy, gastrointestinal acute radiation syndrome, cardiovascular acute radiation syndrome, or central nervous system (CNS) acute radiation syndrome, or combination thereof.

4. 1-palmitoyl-2-linoleoyl-3-acetylglycerol (PLAG) for use according to any one of claims 1 through 3, wherein the PLAG is first administered to the subject within 36 hours after radiation exposure.

5. 1-palmitoyl-2-linoleoyl-3-acetylglycerol (PLAG) for use according to any one of claims 1 through 4, wherein the PLAG is administered with G-CSF.

6. 1-palmitoyl-2-linoleoyl-3-acetylglycerol (PLAG) for use according to any one of claims 1 through 5, wherein the PLAG is administered with one or more selected from the group consisting of pain medicines, antiulcer drugs, antidiarrheal drugs, antibiotics, antifebriles, dietary supplements, and antioxidants.

7. A kit for use in treating or preventing acute radiation syndrome in a subject comprising:
(a) 1-palmitoyl-2-linoleoyl-3-acetylglycerol (PLAG);
(b) instructions for using the PLAG for treating or preventing acute radiation syndrome (ARS) of a subject.

8. A kit for use in treating or preventing exposure to excessive ionizing radiation comprising:
(c) 1-palmitoyl-2-linoleoyl-3-acetylglycerol (PLAG);
(d) instructions for using the PLAG for treating or preventing exposure to excessive ionizing radiation.

9. A kit for use in treating or preventing one or more subsyndromes of acute radiation syndrome comprising:
(e) 1-palmitoyl-2-linoleoyl-3-acetylglycerol (PLAG);
(f) instructions for using the PLAG for treating or preventing one or more subsyndromes of acute radiation syndrome.

10. A kit for use of any one of claims 7 through 9 wherein the kit comprises a therapeutically effective amount of PLAG.

11. A kit for use of any one of claims 7 through 10 wherein the kit comprises written instructions for use of the PLAG.

12. A kit for use of any one of claims 7 through 11 wherein the instructions are a product label.

## Patentansprüche

1. 1-Palmitoyl-2-linoleoyl-3-acetylglycerol (PLAG) zur Verwendung bei der Behandlung oder Vorbeugung des akuten Strahlungssyndroms bei einem Patienten.

2. 1-Palmitoyl-2-linoleoyl-3-acetylglycerol (PLAG) zur Verwendung bei der Behandlung von einem Patienten, der schädlicher ionisierender Strahlung ausgesetzt ist.

3. 1-Palmitoyl-2-linoleoyl-3-acetylglycerin (PLAG) zur Verwendung nach Anspruch 1 oder 2, wobei der Patient an einem hämatopoetischen akuten Strahlungssyndrom, einer strahleninduzierten Koagulopathie, einem gastrointestinalen akuten Strahlungssyndrom, einem kardiovaskulären akuten Strahlungssyndrom oder einem akuten Strahlungssyndrom des zentralen Nervensystems (ZNS) oder einer Kombination davon leidet oder dafür anfällig ist.

4. 1-Palmitoyl-2-linoleoyl-3-acetylglycerin (PLAG) zur Verwendung nach einem der Ansprüche 1 bis 3, wobei das PLAG dem Patienten erstmals innerhalb von 36 Stunden nach der Strahlenbelastung verabreicht wird.

5. 1-Palmitoyl-2-linoleoyl-3-acetylglycerin (PLAG) zur Verwendung nach einem der Ansprüche 1 bis 4, wobei das PLAG mit G-CSF verabreicht wird.

6. 1-Palmitoyl-2-linoleoyl-3-acetylglycerin (PLAG) zur Verwendung nach einem der Ansprüche 1 bis 5, wobei das PLAG mit einem oder mehreren Arzneimitteln, ausgewählt aus der Gruppe bestehend aus Schmerzmitteln, Medikamenten gegen Magengeschwüre, Medikamenten gegen Durchfall, Antibiotika, Medikamenten gegen Durchfall, Nahrungsergänzungsmitteln und Antioxidantien, verabreicht wird.

7. Kit zur Verwendung bei der Behandlung oder Vorbeugung des akuten Strahlungssyndroms bei einem Patienten, umfassend:
(a) 1-Palmitoyl-2-linoleoyl-3-acetylglycerin (PLAG);
(b) Anweisungen zur Verwendung des PLAG zur Behandlung oder Vorbeugung des akuten Strahlensyndroms (ARS) eines Patienten.

8. Kit zur Verwendung bei der Behandlung oder Vorbeugung bei der Exposition gegenüber übermäßiger ionisierender Strahlung, umfassend:
(c) 1-Palmitoyl-2-linoleoyl-3-acetylglycerin (PLAG);
(d) Anweisungen für die Verwendung des PLAG zur Behandlung oder Vorbeugung bei der Exposition gegenüber übermäßiger ionisierender Strahlung.

9. Kit zur Verwendung bei der Behandlung oder Vorbeugung von einem oder mehreren Untersyndromen des akuten Strahlensyndroms, umfassend:
(e) 1-Palmitoyl-2-linoleoyl-3-acetylglycerin (PLAG);
(f) Anweisungen zur Verwendung des PLAG zur Behandlung oder Vorbeugung eines oder mehrerer Untersyndrome des akuten Strahlensyndroms.

10. Kit zur Verwendung nach einem der Ansprüche 7 bis 9, wobei das Kit eine therapeutisch wirksame Menge von PLAG enthält.

11. Kit zur Verwendung nach einem der Ansprüche 7 bis 10, wobei das Kit schriftliche Anweisungen zur Verwendung des PLAG enthält.

12. Kit zur Verwendung nach einem der Ansprüche 7 bis 11, wobei die Anweisungen ein Produktetikett sind.

## Revendications

1. 1-Palmitoyl-2-linoleoyl-3-acétylglycérol (PLAG) pour le traitement ou la prévention du syndrome d'irradiation aiguë chez un sujet.

2. 1-Palmitoyl-2-linoleoyl-3-acétylglycérol (PLAG) pour le traitement d'un sujet exposé à des rayonnements ionisants nocifs.

3. 1-Palmitoyl-2-linoleoyl-3-acétylglycérol (PLAG) pour l'utilisation selon la revendication 1 ou 2, dans laquelle le sujet souffre ou est susceptible de souffrir d'un syndrome d'irradiation hématopoïétique aiguë, d'une coagulopathie radio-induite, d'un syndrome d'irradiation gastro-intestinale aiguë, d'un syndrome d'irradiation cardiovasculaire aiguë ou d'un syndrome d'irradiation du système nerveux central (SNC) aiguë, ou d'une combinaison de ces syndromes.

4. 1-Palmitoyl-2-linoleoyl-3-acetylglycerol (PLAG) pour l'utilisation selon l'une des revendications 1 à 3, dans laquelle le PLAG est administré pour la première fois au sujet dans les 36 heures suivant l'exposition aux radiations.

5. 1-Palmitoyl-2-linoleoyl-3-acétylglycérol (PLAG) pour l'utilisation selon l'une des revendications 1 à 4, dans lequel le PLAG est administré avec du G-CSF.

6. 1-Palmitoyl-2-linoleoyl-3-acétylglycérol (PLAG) pour l'utilisation selon l'une des revendications 1 à 5, dans laquelle le PLAG est administré avec un ou plusieurs médicaments choisis dans le groupe constitué par les analgésiques, les antiulcéreux, les antidiarrhéiques, les antibiotiques, les antifébriles, les compléments alimentaires et les antioxydants.

7. Kit à utiliser pour traiter ou prévenir le syndrome d'irradiation aiguë chez un sujet comprenant:
(a) 1-Palmitoyl-2-linoléoyl-3-acétylglycérol (PLAG);
(b) des instructions concernant l'utilisation du PLAG pour traiter ou prévenir le syndrome d'irradiation aiguë (SIA) d'un sujet.

8. Kit à utiliser pour traiter ou prévenir l'exposition à des rayonnements ionisants excessifs comprenant:
(c) 1-Palmitoyl-2-linoléoyl-3-acétylglycérol (PLAG);
(d) des instructions pour l'utilisation du PLAG dans le traitement ou la prévention de l'exposition à des rayonnements ionisants excessifs.

9. Kit à utiliser pour traiter ou prévenir un ou plusieurs sous-syndromes du syndrome d'irradiation aiguë comprenant:
(e) 1-Palmitoyl-2-linoléoyl-3-acétylglycérol (PLAG);
(f) des instructions concernant l'utilisation du PLAG pour traiter ou prévenir un ou plusieurs sous-syndromes du syndrome d'irradiation aiguë.

10. Kit pour l'utilisation selon l'une des revendications 7 à 9, dans lequel le kit comprend une quantité thérapeutiquement efficace de PLAG.

11. Kit pour l'utilisation selon l'une des revendications 7 à 10, dans lequel le kit comprend des instructions écrites pour l'utilisation du PLAG.

12. Kit pour l'utilisation selon l'une des revendications 7 à 11, dans lequel les instructions sont une étiquette de produit.
